# EUROPEAN PATENT APPLICATION

(11) **EP 4 710 944 A1**
(43) Date of publication of application: **18.03.2026**
(21) Application number: 24803035.5
(22) Date of filing: 09.05.2024
(51) Int. Cl.: A61K 47/68, C07D 491/22, C07K 16/28, A61P 35/00

(54) **ANTI-PDL1 ANTIBODY-DRUG CONJUGATE AND USE**

(30) Priority: 09.05.2023 CN 202310513959; 10.10.2023 CN 202311311898
(71) Applicant: Shanghai Henlius Biotech, Inc., Shanghai 201210 (CN); Shanghai Henlius Biologics Co., Ltd., Shanghai 201616 (CN)
(72) Inventor: SHAN, Yongqiang, Shanghai 201210 (CN); SONG, Hongbin, Shanghai 201210 (CN); SONG, Ge, Shanghai 201210 (CN); JIANG, Jiahao, Shanghai 201210 (CN); ZHANG, Wentao, Shanghai 201210 (CN)
(74) Representative: Plougmann Vingtoft a/s
(86) International application number: PCT/CN2024/091814
(87) International publication number: WO 2024/230755

(57) **Abstract**

An antibody-drug conjugate (ADC) and the use thereof. The present invention relates to a novel anti-PDL1 ADC and a method and use thereof for treating diseases or disorders related to PDL1 expression.

## Description

The present application claims priority to Chinese Patent Application No. CN 202310513959.4 filed on May 9, 2023 and Chinese Patent Application No. CN 202311311898.X filed on October 10, 2023.

### Technical Field

The present application belongs to the pharmaceutical field and relates to an antibody-drug conjugate (ADC) and the use thereof. In particular, the present application relates to a novel anti-PDL1 antibody-drug conjugate, and a method of using same and the use thereof in the treatment of diseases or disorders related to PDL1 expression.

### Background Art

In recent decades, PD-1 and PDL1 antibodies, representative of immune checkpoint inhibitors, have promoted the rapid development of tumor immunotherapy and have become another effective therapeutic means for tumors following surgery, radiotherapy, chemotherapy, and targeted therapy. However, due to the heterogeneity of tumors and the complexity of the regulatory mechanisms of PDL1 expression, as well as the relatively poor permeability of macromolecular anti-PDL1 monoclonal antibodies to dense tumors, the clinical use of anti-PDL1 monoclonal antibodies is greatly limited, and the overall objective response rate in patients is around 15%-30%. There is a significant unmet clinical need.

In order to improve responsiveness and therapeutic effectiveness in patients with tumors, exploration has begun to combine anti-PD-1 monoclonal antibodies with chemotherapy, targeted therapy, and radiation therapy, especially with chemotherapy. Since chemotherapy may cause immunogenic cell death, more tumor antigens can be released, which effectively promotes the activation of the immune response. In addition, the number and activity of immunosuppressive cells can also be reduced. Therefore, chemotherapy is the main alternative solution for immunization combinations. However, systemic side effects caused by chemotherapy remain a very tough problem. PDL1 is expressed in various tumor types and shows limited expression in normal tissues. Therefore, conjugating highly active cytotoxic small molecule chemical drugs with anti-PDL1 monoclonal antibodies can result in bifunctional PDL1-targeted ADC drugs, and this will be a very promising direction in terms of drug development.

Currently, there have been PDL1-targeted ADC drugs in preclinical and phase I trial exploratory phases. SGN-PDL1V is a classic PDL1-targeted ADC platform technical product developed by Seagen, using MMAE as a loaded drug (Payload), and is currently in clinical phase I. MMAE (monomethylauristatin E), one of the most widely used loaded drugs in ADCs, kills cancer cells mainly by inhibiting tubulin polymerization, which is effective in mitotic inhibition; however, the ADC product has a relatively narrow quality window. Therefore, there is an urgent need in the art for drugs that are more biologically active and less toxic, especially drugs that are efficacious for resistant mutants, for example, antibody-drug conjugates, so as to further improve efficacy and reduce side effects.

### Summary of the Invention

The present application relates to an antibody-drug conjugate of formula (I) as defined herein and a pharmaceutical composition comprising the antibody-drug conjugate. The present application is characterized by a method for treating or preventing a tumor or cancer related to PDL1 expression, comprising administering to a subject in need thereof a therapeutically effective amount of the antibody-drug conjugate of formula (I), the prodrug, pharmaceutically acceptable salt, or solvate thereof, or the solvate of the pharmaceutically acceptable salt, as defined herein. The method of the present application can be used for treating or preventing a tumor or cancer related to PDL1 expression.

A first aspect of the present application relates to an antibody-drug conjugate, a prodrug, pharmaceutically acceptable salt, or solvate thereof, or a solvate of the pharmaceutically acceptable salt, wherein the antibody-drug conjugate has a structure of formula (I):

TP-[L1-L2-L3-D]ₖ (I)

wherein
TP is a targeting moiety selectively binding or recognizing PDL1;
L1 is an extension unit connecting TP and L2;
L2 is an optional amino acid residue or a short peptide consisting of 2-10 amino acid residues;
L3 is a spacer element;
D is a biologically active molecule; and
k represents any numerical value between about 0.1 and about 10.0.

Another aspect of the present application relates to a pharmaceutical composition comprising the antibody-drug conjugate of formula (I), the prodrug, pharmaceutically acceptable salt, or solvate thereof, or the solvate of the pharmaceutically acceptable salt, and optionally, a pharmaceutically acceptable carrier, diluent, or excipient.

A further aspect of the present application relates to a method for treating a tumor or cancer related to PDL1 expression. The method comprises administering to a subject in need thereof the antibody-drug conjugate of formula (I), the prodrug, pharmaceutically acceptable salt, or solvate thereof, or the solvate of the pharmaceutically acceptable salt, or a pharmaceutical composition comprising same. The tumor or cancer related to PDL1 expression is selected from melanoma, lung cancer (e.g., non-small cell lung cancer or small cell lung cancer), head and neck cancer, breast cancer (e.g., triple negative breast cancer (TNBC)), ovarian cancer, urothelial cancer, hepatocellular carcinoma (HCC), gastric cancer, cervical cancer, colon cancer, rectal cancer, colorectal cancer, bladder cancer, esophageal cancer, kidney cancer, prostate cancer, pancreatic cancer, and glioma.

A further aspect of the present application relates to the use of the antibody-drug conjugate of formula (I), the prodrug, pharmaceutically acceptable salt, or solvate thereof, or the solvate of the pharmaceutically acceptable salt according to the present invention, or the pharmaceutical composition comprising same in the preparation of a medicament.

In particular, the present application further provides an antibody-drug conjugate and composition with improved potency and/or anti-tumor activity and lower toxicity as compared with anti-PDL1-ADCs known in the prior art.

The details of the present application are set forth in the accompanying description below. Although methods and materials similar or equivalent to those described herein may be used in the practice or testing of the present application, illustrative methods and materials are now described. Other features, objects, and advantages of the present application will be apparent from the description and the claims. In the description and the appended claims, singular forms also include plural forms, unless the context clearly dictates otherwise. Unless otherwise defined, all technical and scientific terms used herein have the same meanings as commonly understood by those of ordinary skill in the field to which the present application belongs. All patents and publications cited in the description are incorporated herein by reference in their entirety.

The contents of all references (including literature references, granted patents, published patent applications, and co-pending patent applications) cited throughout the present application are expressly incorporated herein by reference in their entirety. Unless defined otherwise, all technical and scientific terms used herein have the meanings as commonly understood by those of ordinary skill in the art.

### Brief Description of the Drawings

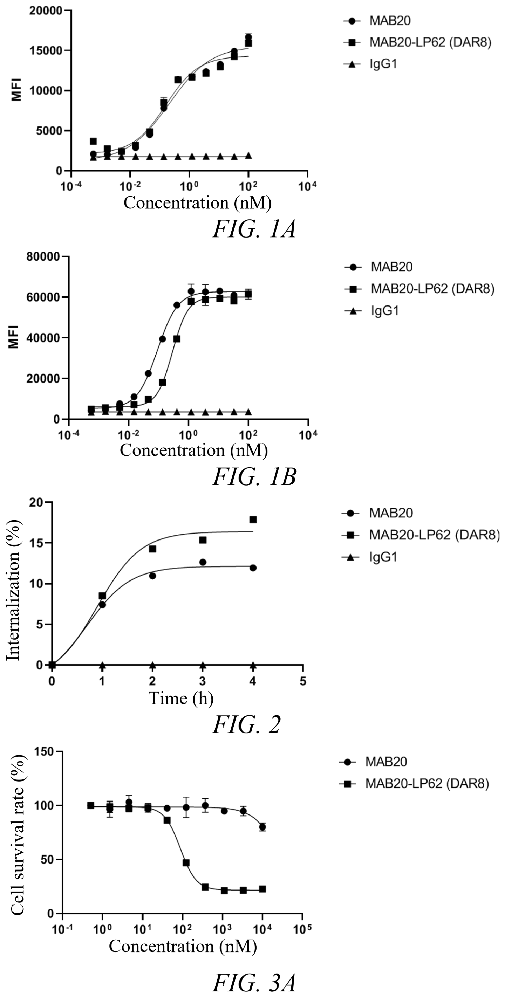

FIG. 1 is a graph showing the affinity of an anti-PDL1 antibody-drug conjugate for PDL1-positive cells NCI-H292 and NCI-H441. (A) Affinity of anti-PDL1 antibody-drug conjugate MAB20-LP62 (DAR8) for NCI-H292 cells. (B) Affinity of anti-PDL1 antibody-drug conjugate MAB20-LP62 (DAR8) for NCI-H441 cells. Note: MAB20 represents an anti-PDL1 antibody, the amino acid sequence of which is set forth in the Sequence Listing; MAB20-LP62 represents an antibody-drug conjugate formed by conjugation of MAB20 to LP62 (the structure of which is shown in the Examples); DAR represents the loaded drug : antibody ratio; IgG1 represents a human IgG1, κ isotype control.
FIG. 2 is a graph showing the internalization of the anti-PDL1 antibody-drug conjugate into PDL1-positive cells NCI-H292 over time. Note: MAB20 represents an anti-PDL1 antibody, the amino acid sequence of which is set forth in the Sequence Listing; MAB20-LP62 represents an antibody-drug conjugate formed by conjugation of MAB20 to LP62; DAR represents the loaded drug : antibody ratio.

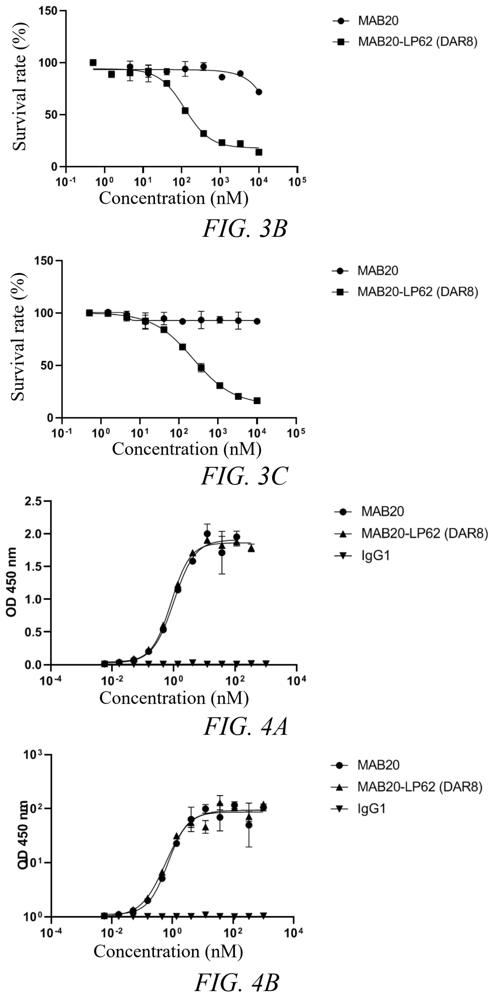

FIG. 3 is a graph showing the cell killing activity of the anti-PDL1 antibody-drug conjugate MAB20-LP62 (DAR8). (A) Cell killing activity of MAB20-LP62 (DAR8) on an NCI-H292 cell line. (B) Cell killing activity of MAB20-LP62 (DAR8) on an BXPC3 cell line. (C) Cell killing activity of MAB20-LP62 (DAR8) on an NCI-H441 cell line. Note: MAB20 represents an anti-PDL1 antibody, the amino acid sequence of which is set forth in the Sequence Listing; MAB20-LP62 represents an antibody-drug conjugate formed by conjugation of MAB20 to LP62; DAR represents the loaded drug : antibody ratio.

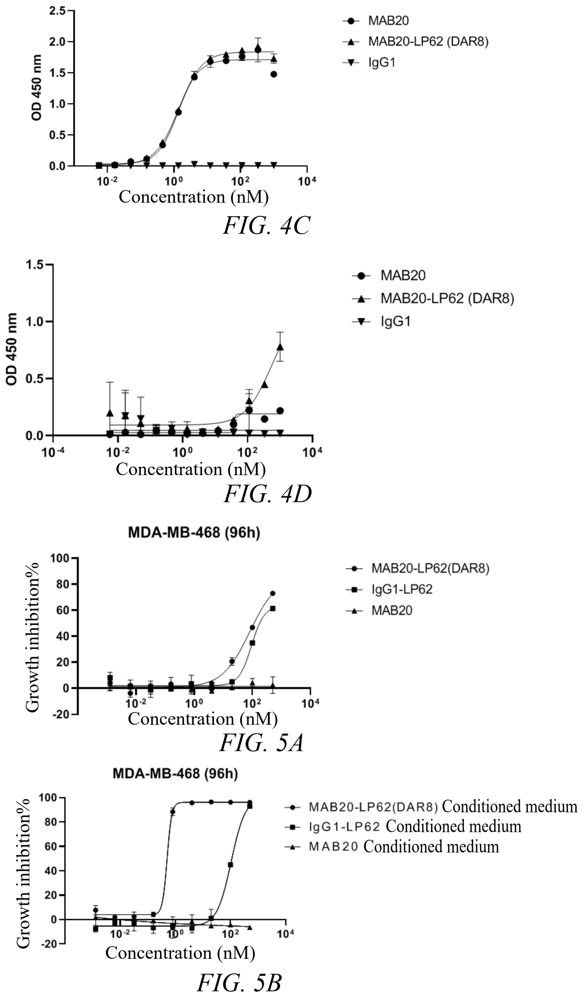

FIG. 4 is a graph showing the cross-binding activity of the anti-PDL1 antibody-drug conjugate to PDL1s derived from different species. (A) MAB20-LP62 (DAR8) binding to human PDL1 ECD. (B) MAB20-LP62 (DAR8) binding to cynomolgus PDL1 ECD. (C) MAB20-LP62 (DAR8) binding to mouse PDL1 ECD. (D) MAB20-LP62 (DAR8) binding to rat PDL1 ECD. Note: MAB20 represents an anti-PDL1 antibody, the amino acid sequence of which is set forth in the Sequence Listing; MAB20-LP62 represents an antibody-drug conjugate formed by conjugation of MAB20 to LP62; DAR represents the loaded drug : antibody ratio; IgG1 represents a human IgG1, κ isotype control.
FIG. 5 is a graph showing the bystander effect of the anti-PDL1 antibody-drug conjugate MAB20-LP62 (DAR8). (A) In vitro killing of MDA-MB-468 cells by test molecules. (B) Bystander killing of MDA-MB-468 cells by a supernatant from a medium having PD-L1-overexpressing HEK293 cells incubated with the test molecules. Note: MAB20 represents an anti-PDL1 antibody, the amino acid sequence of which is set forth in the Sequence Listing; MAB20-LP62 represents an antibody-drug conjugate formed by conjugation of MAB20 to LP62; IgG1-LP62 represents an antibody-drug conjugate formed by conjugation of IgG1 to LP62, with a DAR value of 8; DAR represents the loaded drug : antibody ratio.

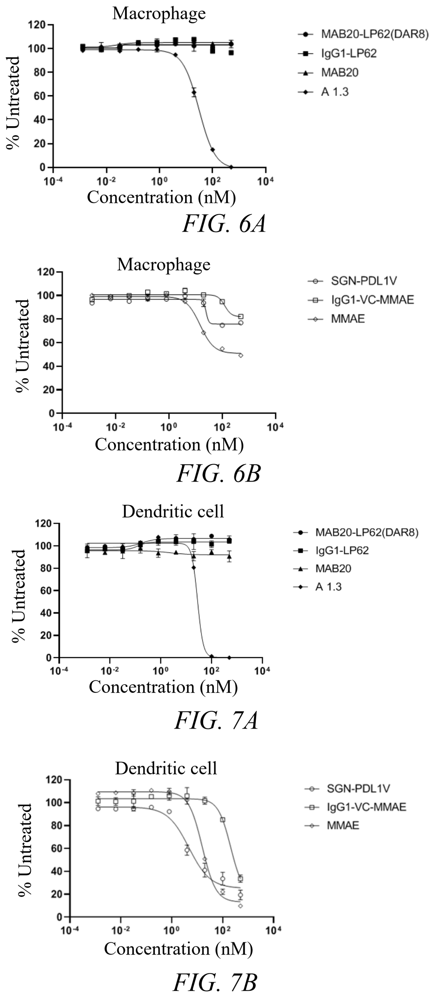

FIG. 6 is a graph showing killing of human macrophages in vitro by anti-PDL1 antibody-drug conjugate MAB20-LP62 (DAR8). Note: MAB20 represents an anti-PDL1 antibody, the amino acid sequence of which is set forth in the Sequence Listing; MAB20-LP62 represents an antibody-drug conjugate formed by conjugation of MAB20 to LP62; IgG1-LP62 represents an antibody-drug conjugate formed by conjugation of IgG1 to LP62; IgG1-VC-MMAE represents an antibody-drug conjugate formed from an isotype control IgG1 prepared in a similar manner to Example 3.2 with MC-VC-PABC-MMAE; SGN-PDL1V represents a reference ADC molecule as described in WO 2021067776 A1, which is an antibody-drug conjugate formed by conjugation of an SGN-PDL1V antibody moiety (the amino acid sequence of which is shown in the Sequence Listing) to a linker-toxin MC-VC-PABC-MMAE; and DAR represents the loaded drug : antibody ratio.
FIG. 7 is a graph showing in vitro killing of human dendritic cells by anti-PDL1 antibody-drug conjugate MAB20-LP62 (DAR8). Note: MAB20 represents an anti-PDL1 antibody, the amino acid sequence of which is set forth in the Sequence Listing; MAB20-LP62 represents an antibody-drug conjugate formed by conjugation of MAB20 to LP62; IgG1-VC-MMAE represents an antibody-drug conjugate formed from an isotype control IgG1 prepared in a similar manner to Example 3.2 with MC-VC-PABC-MMAE; SGN-PDL1V represents a reference ADC molecule as described in WO 2021067776 A1, which is an antibody-drug conjugate formed by conjugation of an SGN-PDL1V antibody moiety (the amino acid sequence of which is shown in the Sequence Listing) to a linker-toxin MC-VC-PABC-MMAE; and DAR represents the loaded drug : antibody ratio.

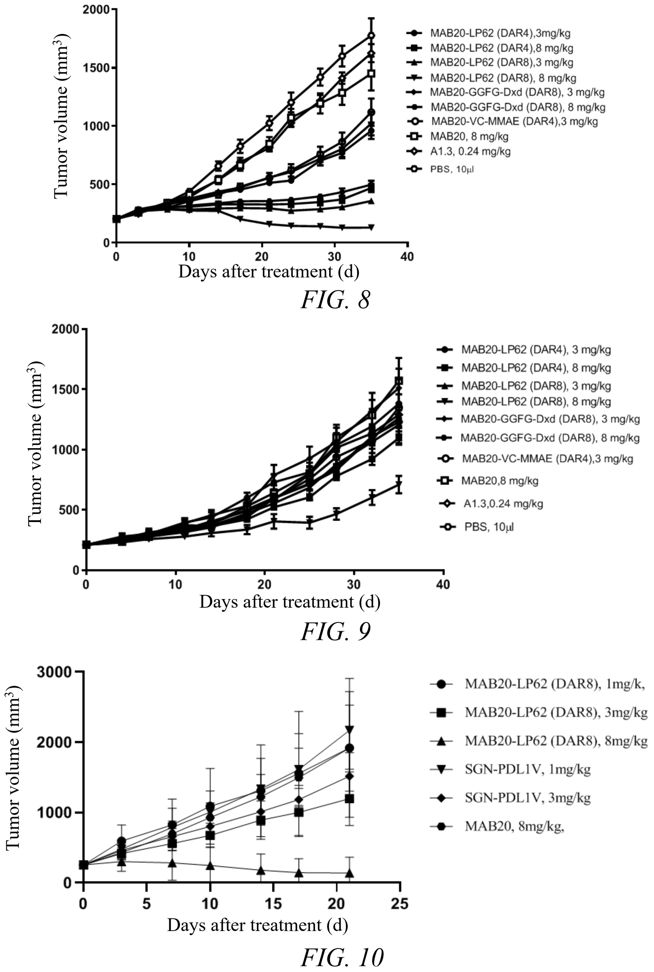

FIG. 8 is a graph showing the anti-tumor activity of the anti-PDL1 antibody-drug conjugate in a NCG mouse model subcutaneously inoculated with the human breast cancer cell line MDA-MB-231. Note: MAB20 represents an anti-PDL1 antibody, the amino acid sequence of which is set forth in the Sequence Listing; MAB20-LP62 represents an antibody-drug conjugate formed by conjugation of MAB20 to LP62; MAB20-VC-MMAE represents an antibody-drug conjugate formed by conjugation of MAB20 to linker-toxin MC-VC-PABC-MMAE; MAB20-GGFG-Dxd represents an antibody-drug conjugate formed by conjugation of MAB20 to linker-toxin MC-GGFG-Dxd; A 1.3 represents toxin compound A 1.3 of Example 1, which is a free loaded drug; DAR represents the loaded drug : antibody ratio.
FIG. 9 is a graph showing the anti-tumor activity of the anti-PDL1 antibody-drug conjugate in a NCG mouse model subcutaneously inoculated with the human pancreatic cancer cell line BxPC3. Note: MAB20 represents an anti-PDL1 antibody, the amino acid sequence of which is set forth in the Sequence Listing; MAB20-LP62 represents an antibody-drug conjugate formed by conjugation of MAB20 to LP62; MAB20-VC-MMAE represents an antibody-drug conjugate formed by conjugation of MAB20 to linker-toxin MC-VC-PABC-MMAE; MAB20-GGFG-DXD represents an antibody-drug conjugate formed by conjugation of MAB20 to linker-toxin MC-GGFG-DXD; A 1.3 represents toxin compound A 1.3 of Example 1, which is a free loaded drug; DAR represents the loaded drug : antibody ratio.
FIG. 10 is a graph showing the anti-tumor effect of the anti-PDL1 antibody-drug conjugate in human non-small cell lung cancer LU6437 PDX xenograft BALB/c nude mice. Note: MAB20 represents an anti-PDL1 antibody, the amino acid sequence of which is set forth in the Sequence Listing; MAB20-LP62 represents an antibody-drug conjugate formed by conjugation of MAB20 to LP62; SGN-PDL1V represents a reference ADC molecule as described in WO 2021067776 A1, which is an antibody-drug conjugate formed by conjugation of an SGN-PDL1V antibody moiety (the amino acid sequence of which is shown in the Sequence Listing) to a linker-toxin MC-VC-PABC-MMAE; and DAR represents the loaded drug : antibody ratio.

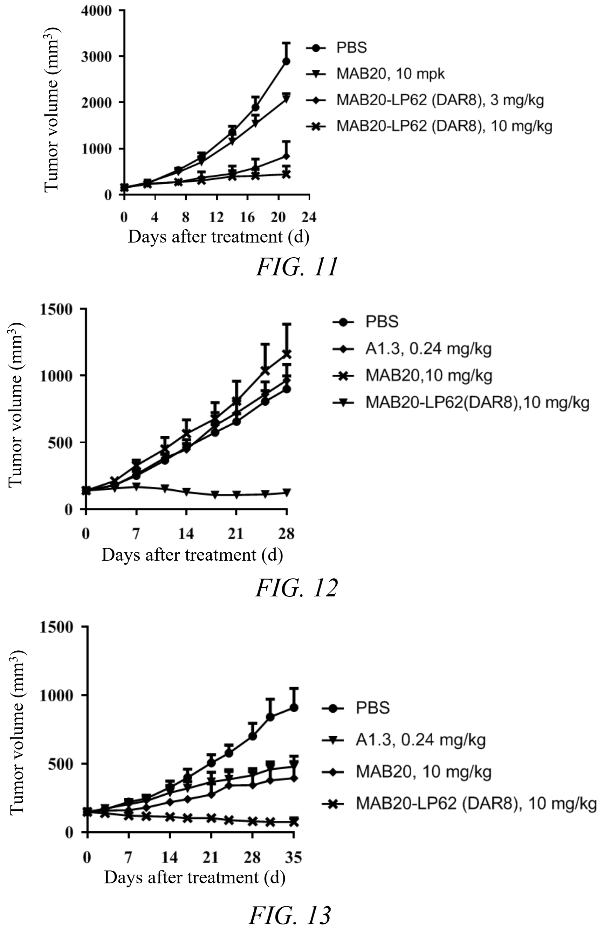

FIG. 11 is a graph showing the anti-tumor effect of the anti-PDL1 antibody-drug conjugate in human PD-L1-positive liver cancer LD1-0011-200617 PDX xenograft NU/NU mice. Note: MAB20 represents an anti-PDL1 antibody, the amino acid sequence of which is set forth in the Sequence Listing; MAB20-LP62 represents an antibody-drug conjugate formed by conjugation of MAB20 to LP62; DAR represents the loaded drug : antibody ratio.
FIG. 12 is a graph showing the anti-tumor effect of the anti-PDL1 antibody-drug conjugate in human PD-L1-positive head and neck squamous carcinoma LD1-2023-411020 PDX xenograft NCG nude mice. Note: MAB20 represents an anti-PDL1 antibody, the amino acid sequence of which is set forth in the Sequence Listing; MAB20-LP62 represents an antibody-drug conjugate formed by conjugation of MAB20 to LP62; A 1.3 represents toxin compound A 1.3 of Example 1, which is a free loaded drug; DAR represents the loaded drug : antibody ratio.
FIG. 13 is a graph showing the anti-tumor effect of the anti-PDL1 antibody-drug conjugate in human PD-L1-positive cervical cancer LD1-0010-200614 PDX xenograft Balb/c nude mice. Note: MAB20 represents an anti-PDL1 antibody, the amino acid sequence of which is set forth in the Sequence Listing; MAB20-LP62 represents an antibody-drug conjugate formed by conjugation of MAB20 to LP62; A 1.3 represents toxin compound A 1.3 of Example 1, which is a free loaded drug; DAR represents the loaded drug : antibody ratio.

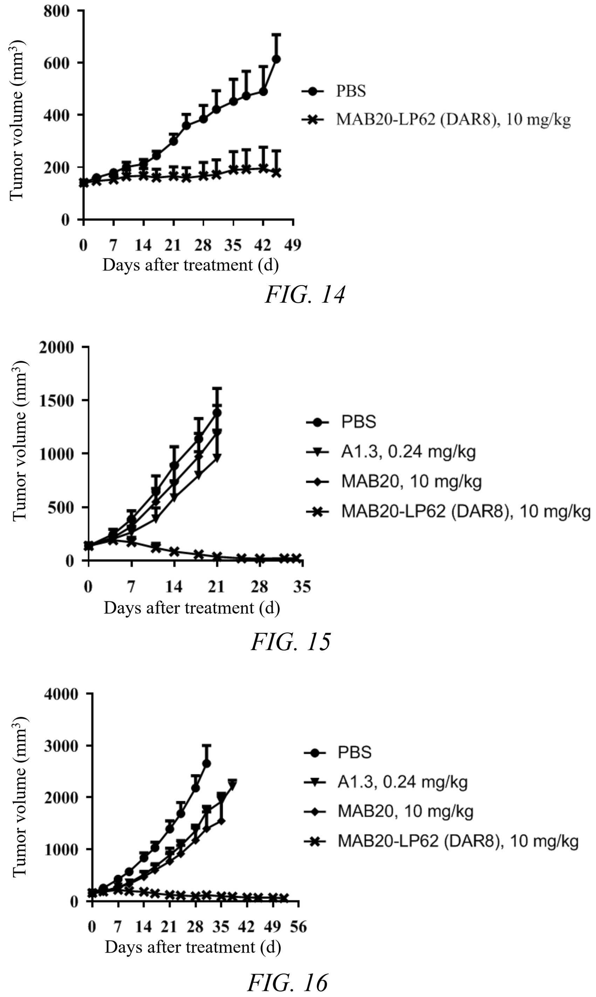

FIG. 14 is a graph showing the anti-tumor effect of the anti-PDL1 antibody-drug conjugate in human PD-L1-positive esophageal squamous carcinoma LD1-0015-362448 PDX xenograft Balb/c nude mice. Note: MAB20 represents an anti-PDL1 antibody, the amino acid sequence of which is set forth in the Sequence Listing; MAB20-LP62 represents an antibody-drug conjugate formed by conjugation of MAB20 to LP62; DAR represents the loaded drug : antibody ratio.
FIG. 15 is a graph showing the anti-tumor effect of the anti-PDL1 antibody-drug conjugate in human PD-L1-positive gastric cancer LD1-0017-200636 PDX xenograft NU/NU mice. Note: MAB20 represents an anti-PDL1 antibody, the amino acid sequence of which is set forth in the Sequence Listing; MAB20-LP62 represents an antibody-drug conjugate formed by conjugation of MAB20 to LP62; A 1.3 represents toxin compound A 1.3 of Example 1, which is a free loaded drug; DAR represents the loaded drug : antibody ratio.
FIG. 16 is a graph showing the anti-tumor effect of the anti-PDL1 antibody-drug conjugate in human PD-L1-positive colorectal cancer LD1-2013-362125 PDX xenograft NU/NU mice. Note: MAB20 represents an anti-PDL1 antibody, the amino acid sequence of which is set forth in the Sequence Listing; MAB20-LP62 represents an antibody-drug conjugate formed by conjugation of MAB20 to LP62; A 1.3 represents toxin compound A 1.3 of Example 1, which is a free loaded drug; DAR represents the loaded drug : antibody ratio.

### Detailed Description of the Invention

The present invention provides a novel anti-PDL1 antibody-drug conjugate. The anti-PDL1 antibody-drug conjugate exhibits good affinity for PDL1-positive cells, can be effectively internalized into the PDL1-positive cells, and also exhibits good cell killing effects even on anti-PDL1 insensitive tumor cells. Furthermore, the anti-PDL1 antibody-drug conjugate has a good bystander effect and lower toxicity (especially immunotoxicity). Furthermore, the anti-PDL1 antibody-drug conjugate exhibits excellent tumor inhibitory activity in a variety of tumor models, even low-PDL1-expressing tumor models.

For the sake of clarity and not as a limitation, the detailed description of the present invention is divided into the following subsections:
1. Definitions;
2. Antibody-drug conjugate;
3. Method of use;
4. Pharmaceutical formulation; and
5. Article of manufacture and kit.

### 1. Definitions

In the present invention, the scientific and technical terms used herein have the meanings as commonly understood by those skilled in the art unless otherwise specified. In addition, the terms and laboratory operation steps related to the protein and nucleic acid chemistry, molecular biology, cell and tissue culture, microbiology and immunology used herein are all terms and conventional steps that are widely used in the corresponding art. Moreover, for better understanding of the present invention, definitions and explanations of related terms are provided below.

The terms "PD-L1", "PDL1", "CD274", "B7-H1", and "programmed cell death ligand 1" are used interchangeably herein and, unless otherwise specified, include any variants, isoforms, and homologs of human PD-L1 that are generally expressed by cells or on cells transfected with the PD-L1 gene.

The term "antibody" is used in its broadest sense and encompasses various antibody structures, including but not limited to monoclonal antibodies, polyclonal antibodies, multispecific antibodies (e.g., bispecific antibodies), full-length antibodies, and antigen-binding fragments thereof, as long as they exhibit the desired antigen-binding activity. The term "antibody moiety" refers to a full-length antibody or an antigen-binding fragment thereof.

The full-length antibody comprises two heavy chains and two light chains. The variable regions of the heavy chains and light chains are responsible for antigen binding. The variable domains of a heavy chain and a light chain may be referred to as "V_{H}" and "V_{L}", respectively. The variable regions in the two chains generally contain three highly variable loops termed complementarity determining regions (CDRs) (light chain (LC) CDRs including LC-CDR1, LC-CDR2, and LC-CDR3, and heavy chain (HC) CDRs including HC-CDR1, HC-CDR2, and HC-CDR3). The CDR boundaries of the antibodies and antigen-binding fragments disclosed herein can be defined or identified by the following conventions: Kabat, Chothia, or Al-Lazikani (Al-Lazikani 1997; Chothia 1985; Chothia 1987; Chothia 1989; Kabat 1987; and Kabat 1991). The three CDRs of the heavy chain or light chain are interposed between flanking segments termed framework regions (FRs), which are more highly conserved than the CDRs and form a scaffold that supports the hypervariable loops. The constant regions of the heavy chain and light chain are not involved in antigen binding but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, δ, ε, γ, and µ heavy chains, respectively. Several of the major antibody classes are divided into subclasses such as lgG1 (γ1 heavy chain), lgG2 (γ2 heavy chain), lgG3 (γ3 heavy chain), lgG4 (γ4 heavy chain), lgA1 (α1 heavy chain), or lgA2 (α2 heavy chain).

The term "antigen-binding fragment" as used herein refers to an antibody fragment, including, for example, bispecific antibodies, Fab, Fab', F(ab')2, an Fv fragment, a disulfide-stabilized Fv fragment (dsFv), (dsFv)2, bispecific dsFv (dsFv-dsFv'), a disulfide-stabilized bispecific antibody (ds bispecific antibody), single-chain Fv (scFv), an scFv dimer (bivalent diabody), a multispecific antibody consisting of an antibody moiety comprising one or more CDRs, a camelized single domain antibody, a nanobody, a domain antibody, a bivalent domain antibody, or any other antibody fragment that binds to an antigen but does not contain a complete antibody structure. An antigen-binding fragment can bind to the same antigen that the parent antibody or parent antibody fragment (e.g., parent scFv) binds to. In some embodiments, an antigen-binding fragment may comprise one or more CDRs from a particular human antibody, wherein the one or more CDRs are grafted to framework regions from one or more different human antibodies.

As used herein, the term "CDR" or "complementarity determining region" is intended to mean non-contiguous antigen binding sites found within the variable region of a heavy chain and/or light chain polypeptide. These specific regions have been described in: Kabat et al., J. Biol. Chem., 252:6609-6616 (1977); Kabat et al., the U.S. Department of Health and Human Services, "Sequences of proteins of immunological interest" (1991); Chothia et al., J. Mol. Biol. 196:901-917 (1987); Al-Lazikani B. et al., J. Mol. Biol., 273: 927-948 (1997); MacCallum et al., J. Mol. Biol. 262:732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309:657-670 (2001), in which the definition includes overlaps or subsets of amino acid residues upon mutual alignment. Nevertheless, application of any one of the definitions to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. CDR prediction algorithms and interfaces are known in the art, including, for example, Abhinandan and Martin, Mol. Immunol., 45: 3832-3839 (2008); Ehrenmann F. et al., Nucleic Acids Res., 38: D301-D307 (2010); and Adolf-Bryfogle J. et al., Nucleic Acids Res., 43: D432-D438 (2015). The contents of the references cited in this paragraph are incorporated herein by reference in their entireties for use in the present application and for possible inclusion in one or more claims herein.

**Table 1: CDR definitions**

| | **Kabat¹** | **Chothia²** | **MacCallum³** | **IMGT⁴** | **AHo⁵** |
|---|---|---|---|---|---|
| V_{H} CDR1 | 31-35 | 26-32 | 30-35 | 27-38 | 25-40 |
| V_{H} CDR2 | 50-65 | 53-55 | 47-58 | 56-65 | 58-77 |
| V_{H} CDR3 | 95-102 | 96-101 | 93-101 | 105-117 | 109-137 |
| V_{L} CDR1 | 24-34 | 26-32 | 30-36 | 27-38 | 25-40 |
| V_{L} CDR2 | 50-56 | 50-52 | 46-55 | 56-65 | 58-77 |
| V_{L} CDR3 | 89-97 | 91-96 | 89-96 | 105-117 | 109-137 |

| | | | | | |
|---|---|---|---|---|---|
| ¹Residue numbering follows the nomenclature of Kabat et al., supra. ²Residue numbering follows the nomenclature of Chothia et al., supra. ³Residue numbering follows the nomenclature of MacCallum et al., supra. ⁴Residue numbering follows the nomenclature of Lefranc et al., supra. ⁵Residue numbering follows the nomenclature of Honegger and Plückthun, supra. | | | | | |

The expression "variable domain residue numbering as in Kabat" or "amino acid position numbering as in Kabat", and variations thereof, refer to a numbering system used for heavy chain variable domains or light chain variable domains from antibody compilation by Kabat et al., supra. Using this numbering system, the actual linear amino acid sequence may contain shortened or inserted fewer or additional amino acids corresponding to an FR or hypervariable region (HVR) of a variable domain. For example, a heavy chain variable domain may comprise a single amino acid insertion (residue 52a according to Kabat) after residue 52 of H2 and inserted residues (e.g., residues 82a, 82b, and 82c according to Kabat) after heavy chain FR residue 82. The Kabat numbering of residues in a given antibody can be determined by aligning the sequence of the antibody with a "standard" Kabat-numbered sequence across regions of homology.

Unless otherwise specified herein, amino acid residues encompassing the CDRs of a full-length antibody are defined according to the Kabat nomenclature of Kabat et al., supra, and the numbering of residues in an immunoglobulin heavy chain, e.g., an Fc region, refers to numbering by the EU index as described in Kabat et al., supra, except that the amino acid residues of CDRs encompassing any consensus sequence are defined according to the Kabat nomenclature, wherein modifications are based on experimental conditions. The "EU index as described in Kabat" refers to the residue numbering of a human IgG1 EU antibody.

"Framework" or "FR" residues are those variable domain residues other than the CDR residues as defined herein.

A "humanized" form of a non-human (e.g., rodent) antibody is a chimeric antibody that contains a minimal sequence derived from a non-human antibody. In most cases, a humanized antibody is a human immunoglobulin (receptor antibody) in which residues from the hypervariable region (HVR) of a recipient are replaced by residues from a hypervariable region (donor antibody) with the desired antigen specificity, affinity, and capacity in a non-human species (e.g., a mouse, a rat, a rabbit, or a non-human primate). In some examples, framework region (FR) residues of a human immunoglobulin are replaced by corresponding non-human residues. In addition, a humanized antibody can comprise residues not found in the recipient antibody or the donor antibody. These modifications are made to further improve the performance of the antibody. In general, a humanized antibody will comprise at least one, and typically substantially both, of the two variable domains, wherein all or substantially all of the hypervariable loops correspond to those of a non-human immunoglobulin, and all or substantially all of the FRs are those of the human immunoglobulin sequence. A humanized antibody will also optionally comprise at least a portion of an immunoglobulin constant region (Fc), typically at least a portion of a human immunoglobulin. For further details, see Jones et al., Nature, 321: 522-525, (1986); Riechmann et al., Nature, 332:323-329 (1988); and Presta, Curr. Op. Struct. Biol. 2:593-596 (1992).

A "human antibody" is an antibody that has an amino acid sequence corresponding to that of an antibody produced in a human and/or has been made using any technique as disclosed herein for making human antibodies. This definition of a human antibody specifically excludes a humanized antibody comprising non-human antigen-binding residues. Human antibodies can be produced using various techniques known in the art, including phage display libraries. Hoogenboom and Winter, J. Mol. Biol., 227:381 (1991); and Marks et al., J. Mol. Biol., 222:581 (1991). Also useful for the preparation of human monoclonal antibodies are methods described in Cole et al., Monoclonal Antibodies and Cancer Therapy, Alan R. Liss, p. 77 (1985); and Boerner et al., J. Immunol., 147(1):86-95 (1991). See also van Dijk and van de Winkel, Curr.Opin. Pharmacol., 5: 368-74 (2001). Human antibodies can be prepared by administering an antigen to a transgenic animal that has been modified to produce such antibodies in response to antigenic challenge, but whose endogenous loci have been disabled, e.g., immunized xenomice (see, e.g., U.S. Patent Nos. 6,075,181 and 6,150,584 for XENOMOUSE^{™} technology). See also, for example, Li et al., Proc. Natl. Acad. Sci. USA 103:3557-3562 (2006) for human antibodies generated via human B cell hybridoma technology.

"Percent (%) amino acid sequence identity" or "homology" with respect to the polypeptide and antibody sequences identified herein is defined as the percentage of amino acid residues in a candidate sequence that are identical to the amino acid residues in the polypeptide being compared, after sequence alignment (considering any conservative substitutions as part of the sequence identity). Alignment for purposes of determining percent amino acid sequence identity can be achieved by using various methods within techniques in the art, for instance, by using publicly available computer software such as BLAST, BLAST-2, ALIGN, Megalign (DNASTAR), or MUSCLE software. Those skilled in the art can determine appropriate parameters for measuring alignment, including any algorithms needed to achieve maximal alignment over the full-length of the sequences being compared. However, for purposes herein, % amino acid sequence identity values are generated using the sequence alignment computer program MUSCLE (Edgar, R.C., Nucleic Acids Research 32(5):1792-1797, 2004; and Edgar, R.C., BMC Bioinformatics 5(1):113, 2004).

"Homologous" refers to the sequence similarity or sequence identity between two polypeptides or between two nucleic acid molecules. When a position in both of two sequences being compared is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of the positions being compared times 100. For example, if 6 of 10 positions in two sequences are matched or homologous, the two sequences are 60% homologous. By way of example, the DNA sequences ATTGCC and TATGGC share 50% homology. Generally, comparison is made when two sequences are aligned to give maximum homology.

The term "constant domain" refers to a portion of an immunoglobulin molecule that has a more conserved amino acid sequence relative to the other portion of the immunoglobulin, i.e., a variable domain, which contains an antigen-binding site. A constant domain contains C_{H}1, C_{H}2, and C_{H}3 domains (collectively referred to as C_{H}) of a heavy chain and a CHL (or C_{L}) domain of a light chain.

A "light chain" of an antibody (immunoglobulin) from any mammalian species can be assigned to one of two clearly distinct types, called kappa ("x") and lambda ("λ"), respectively, based on the amino acid sequence of the constant domain thereof.

The "CH1 domain" (also referred to as "C1" of "H1" domain) is usually from about amino acid 118 to about amino acid 215 (EU numbering system).

A "hinge region" is generally defined as a region in IgG that corresponds to Glu216 to Pro230 of human IgG1 (Burton, Molec. Immunol., 22:161-206 (1985)). Hinge regions of other IgG isotypes may be aligned with an IgG1 sequence by placing the first and last cysteine residues forming inter-heavy-chain S-S bonds in the same positions.

The "CH2 domain" of a human IgG Fc region (also referred to as "C2" domain) is usually from about amino acid 231 to about amino acid 340. The CH2 domain is unique in that it is not closely paired with another domain. Instead, two N-linked branched carbohydrate chains are interposed between the two CH2 domains of an intact native IgG molecule. It has been speculated that the carbohydrate may provide a substitute for domain-domain pairing and help stabilize the CH2 domain. Burton, Molec Immunol., 22:161-206 (1985).

The "CH3 domain" (also referred to as "C2" domain) comprises a residue region in the Fc region proximal to the C-terminus of the CH2 domain (i.e., from about amino acid residue 341 to the C-terminus of the antibody sequence (typically at residue 446 or 447 in IgG)).

The term "Fc region" or "fragment crystallizable region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to extend from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may include antibody populations from which all K447 residues have been removed, antibody populations from which no K447 residues have been removed, and antibody populations having a mixture of antibodies with and without the K447 residue. Suitable native sequence Fc regions for use in the antibodies described herein include human IgG1, IgG2 (IgG2A, IgG2B), IgG3, and IgG4.

"Fc receptor" or "FcR" describes a receptor that binds the Fc region of an antibody. A preferred FcR is a native human FcR. Moreover, a preferred FcR is one which binds an IgG antibody (γ receptor) and includes receptors of the FcγRI, FcγRII, and FcγRIII subclasses, including allelic variants and alternatively spliced forms of these receptors, FcγRII receptors include FcγRIIA ("activating receptor") and FcγRIIB ("inhibitory receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibitory receptor FcγRIIB contains an immunoreceptor tyrosine-based inhibition motif (ITIM) in its cytoplasmic domain. (See M. Daëron, Annu. Rev. Immunol. 15:203-234 (1997). FcR is reviewed in Ravetch and Kinet, Annu. Rev. Immunol. 9: 457-92 (1991); Capel et al., Immunomethods 4: 25-34 (1994); and de Haas et al., J. Lab.Clin. Med. 126: 330-41 (1995). The term "FcR" herein encompasses other FcRs, including those to be identified in the future.

As used herein, the terms "specifically binds", "specifically recognizing", and "is specific for" refer to measurable and reproducible interactions, such as binding between a target and an antibody or antibody moiety, which is determinative of the presence of the target in the presence of a heterogeneous population of molecules, including biological molecules. For example, an antibody or antibody moiety that specifically recognizes a target (which can be an epitope) is an antibody or antibody moiety that binds this target with greater affinity, greater avidity, greater readiness, and/or greater duration than its binding to other targets. In some embodiments, the extent of binding of an antibody to an unrelated target is less than about 10% of the extent of binding of the antibody to the target as measured, e.g., by radioimmunoassay (RIA). In some embodiments, the dissociation constant of an antibody that specifically binds to a target is (K_{D}) ≤ 10⁻⁵ M, ≤ 10⁻⁶ M, ≤ 10⁻⁷ M, ≤ 10⁻⁸ M, ≤ 10⁻⁹ M, ≤ 10⁻¹⁰ M, ≤ 10⁻¹¹ M, or ≤ 10⁻¹² M. In some embodiments, an antibody specifically binds to an epitope of a protein that is conserved among proteins of various species. In some embodiments, specific binding can include, but does not require, exclusive binding. The binding specificity of an antibody or antigen-binding domain can be determined experimentally by a method known in the art. Such methods include, but are not limited to, Western blots, ELISA-, RIA-, ECL-, IRMA-, EIA-, BIACORE^{™}-assays and peptide scans.

As used herein, the term "cytotoxic agent" refers to a substance that inhibits or prevents cell functions and/or causes cell destruction. The term is intended to include: radioisotopes, e.g., the radioisotopes At²¹¹, I¹³¹, I¹²⁵, Y⁹⁰, Re¹⁸⁶, Re¹⁸⁸, Sm¹⁵³, Bi²¹², P³², C⁶⁰, and Lu; chemotherapeutic agents; and toxins, such as small molecule toxins or enzymatic toxins of bacterial, fungal, plant, or animal origin, including synthetic analogs and derivatives thereof.

A "linker", "linker unit", or "linker element" refers to a chemical module comprising a covalent bond or chain of atoms that covalently links an antibody or targeting moiety to a drug module.

As used herein, the terms "antibody-drug conjugate", "antibody conjugate", "conjugate", "immunoconjugate", and "ADC" are used interchangeably and refer to a compound or derivative thereof linked to an antibody, e.g., an anti-PDL1 antibody, as defined by the following general formula: Ab-(L-D)k, in which Ab = antibody moiety (i.e., antibody or antigen-binding fragment), L = linker moiety, D = drug moiety, and k = the number of drug moieties conjugated per antibody moiety.

Among currently commercially available antibody-drug conjugates, there are two main methods for conjugating an antibody to a linker: (1) Lysine is the most common site of attachment in antibodies, and an ε-amino group thereof can react with an activated carboxyl group in the linker to form an amide bond. Currently, there have been techniques that achieve site-directed conjugation by activating a carboxyl group in the linker with an activating group and then forming an amide bond with the ε-amino group of a specific lysine in the antibody. (2) The sulfhydryl groups (SH) of cysteines in an antibody all exist in the form of disulfide bonds (-S-S-). Opening the disulfide bond in the antibody can provide a plurality of free sulfhydryl groups as sites of conjugation. Conjugation to the sulfhydryl groups of the antibody is done either by a Michael addition reaction between a free sulfhydryl group on the antibody and maleimide, or by double Michael addition reactions between a specific substrate and the free sulfhydryl group on the antibody to form a structurally unique sulfur bridge bond.

The three-letter and one-letter codes of amino acids as used herein are as described in J. boil. Chem. 1968, 243, 3558.

As used herein, the term "halogen" refers to fluorine, chlorine, bromine, or iodine.

As used herein, the term "alkyl" refers to a saturated aliphatic hydrocarbon group, which is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferably, the alkyl is a lower alkyl group containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl group may be substituted or unsubstituted. When substituted, the substituent may be substituted at any available point of attachment, and the substituent is preferably one or more groups independently selected from alkyl, alkenyl, alkynyl, alkoxy, alkylthio, alkylamino, halogen, sulfhydryl, hydroxy, nitro, amino, cycloalkyl, heterocycloalkyl, aryl, heteroaryl, cycloalkoxy, heterocycloalkoxy, cycloalkylthio, heterocycloalkylthio, and oxo.

As used herein, the term "haloalkyl" refers to alkyl substituted with one or more halogen, wherein the alkyl is as defined above.

As used herein, the term "cyano" refers to -CN.

As used herein, the term "sulfonic acid group" refers to the group -SO₃H.

As used herein, the term "carboxy" refers to -C(O)OH.

As used herein, the term "alkoxy" refers to -O-(alkyl) and -O-(cycloalkyl), in which the alkyl or cycloalkyl is as defined above. Non-limiting examples of alkoxy include: methoxy, ethoxy, propoxy, butoxy, cyclopropoxy, cyclobutoxy, cyclopentoxy, and cyclohexoxy. The alkoxy can be optionally substituted or unsubstituted, and when it is substituted, the substituent is preferably one or more groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "alkenyl" refers to an alkyl compound containing at least one carbon-carbon double bond in the molecule, wherein the alkyl is as defined above, and the alkenyl has 2 to 10 (e.g., 2, 3, 4, 5, 6, 7, 8, 9, or 10) carbon atoms (i.e., C₂₋₁₀ alkenyl). The alkenyl is preferably an alkenyl group having 2 to 6 carbon atoms (i.e., C₂₋₆ alkenyl). The alkenyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl compound containing at least one carbon-carbon triple bond in the molecule, wherein the alkyl is as defined above, and the alkenyl has 2 to 10 (e.g., 2, 3, 4, 5, 6, 7, 8, 9, or 10) carbon atoms (i.e., C₂₋₁₀ alkynyl). The alkynyl is preferably an alkynyl group having 2 to 6 carbon atoms (i.e., C₂₋₆ alkynyl). The alkynyl may be substituted or unsubstituted. When it is substituted, the substituent is preferably selected from one or more of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

As used herein, the term "deuterated alkyl" refers to alkyl substituted with one or more halogen, wherein the alkyl is as defined above.

The present invention further comprises various deuterated forms of formula (I). Each available hydrogen atom attached to a carbon atom can be independently replaced by a deuterium atom. Those skilled in the art can synthesize deuterated forms of formula (I) by reference to relevant literatures. Commercially available deuterated starting materials may be used in preparing deuterated forms of formula (I), or they may be synthesized using conventional techniques employing deuterated reagents, non-limiting examples of which include: deuterated borane, a solution of a trideuterated borane in tetrahydrofuran, deuterated lithium aluminum hydride, deuterated iodoethane, deuterated iodomethane, etc.

As used herein, the term "DAR" refers to the loaded drug : antibody ratio, which represents the average number of cytotoxic drugs loaded per antibody, or may also represent the ratio of the amount of the drug to the amount of the antibody. The drug loading may range from 0 to 12, preferably 1 to 10 cytotoxic drugs (D) conjugated per antibody (Ab). In an embodiment of the present invention, the DAR is denoted as k, which can be an average value of 1, 2, 3, 4, 5, 6, 7, 8, 9, or 10, by way of example, without limitation. The average number of drugs per ADC molecule after a conjugation reaction can be characterized by conventional methods such as UV/visible spectroscopy, mass spectrometry, ELISA assay, and HPLC.

In one embodiment of the present invention, the cytotoxic drug is conjugated to an exposed interchain cysteine sulfhydryl group (-SH) of an antibody and/or a cysteine sulfhydryl group (-SH) resulting from site-directed mutagenesis via a linker unit. Generally, the number of drug molecules that can be conjugated to the antibody in a conjugation reaction will be less than or equal to the theoretically maximum value.

The following non-limiting methods can be used to control the loading of a ligand-cytotoxic drug conjugate and include:
(1) controlling the molar ratio of a linker-loaded drug to a reducing agent to a monoclonal antibody,
(2) controlling reaction time, temperature, and pH value, and
(3) selecting different reaction reagents and the content thereof.

As used herein, the term "solvated form" or "solvate" means that the ligand-drug conjugate of the present invention forms a pharmaceutically acceptable solvate with one or more solvent molecules, non-limiting examples of which include water, ethanol, acetonitrile, isopropanol, DMSO, ethyl acetate, dimethylacetamide (DMAC), etc.

The phrase "pharmaceutically acceptable salt" as used herein refers to a pharmaceutically acceptable organic or inorganic salt of an antibody-drug conjugate. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tanninate, pantothenate, bitartrate, ascorbate, succinate, maleate, gentisate, fumarate, gluconate, glucuronate, sugar acid salt, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)). Pharmaceutically acceptable salts may involve the inclusion of another molecule, such as an acetate ion, a succinate ion, or other counterions. The counterions can be any organic or inorganic module that stabilizes the charge of the parent compound. In addition, pharmaceutically acceptable salts may have more than one charged atom in their structure. Where a variety of charged atoms act as a constituent of a pharmaceutically acceptable salt, there may be a variety of counterions. Thus, a pharmaceutically acceptable salt may have one or more charged atoms and/or one or more counterions.

A "pharmaceutically acceptable solvate" refers to a combination of one or more solvent molecules and an ADC or a salt thereof. Examples of solvents that form pharmaceutically acceptable solvates include, but are not limited to, water, isopropanol, ethanol, methanol, DMSO, ethyl acetate, acetic acid, and ethanolamine.

As used herein, "treatment" or "treating" is an approach for obtaining beneficial or desired results, including clinical results. For purposes of the present application, the beneficial or desired clinical results include, but are not limited to, one or more of: alleviating one or more symptoms resulting from the disease, diminishing the extent of the disease, stabilizing the disease (e.g., preventing or delaying the worsening of the disease), preventing or delaying the spread (e.g., metastasis) of the disease, preventing or delaying the recurrence of the disease, delaying or slowing the progression of the disease, ameliorating the disease state, providing a remission (partial or total) of the disease, decreasing the dose of one or more other drugs required to treat the disease, delaying the progression of the disease, increasing or ameliorating the quality of life, increasing body weight gain, and/or prolonging survival. Also encompassed by "treatment" is a reduction of pathological consequence of cancer (such as, for example, tumor volume). The method of the present application contemplates any one or more of these aspects of treatment.

In the context of cancer, the term "treatment" includes any one or all of: inhibiting the growth of cancer cells, inhibiting the replication of cancer cells, reducing the overall tumor burden, and ameliorating one or more symptoms related to the disease.

The term "inhibition" or "inhibit" refers to the reduction or cessation of any phenotypic characteristic, or the reduction or cessation of the incidence, extent or likelihood of that characteristic. "Reducing" or "inhibiting" refers to reducing, lowering, or preventing activity, function and/or amount as compared with a reference. In certain embodiments, "reducing" or "inhibiting" refers to the ability to cause an overall reduction of 20% or more. In another embodiment, "reducing" or "inhibiting" refers to the ability to cause an overall reduction of 50% or more. In yet another embodiment, "reducing" or "inhibiting" refers to the ability to cause an overall reduction of 75%, 85%, 90%, 95%, or more.

As used herein, "reference" refers to any sample, standard, or level used for comparison purposes. The reference can be obtained from healthy and/or non-diseased samples. In certain embodiments, the reference may be obtained from an untreated sample. In certain embodiments, the reference is obtained from a non-diseased or untreated sample of an individual. In some examples, the reference is obtained from one or more healthy individuals other than the individual or patient.

As used herein, "delaying the development of a disease" refers to delaying, impeding, slowing, retarding, stabilizing, inhibiting, and/or delaying the development of a disease, such as cancer. The delay may vary in the length of time, depending on the history of the disease and/or the individual being treated. It would be obvious to those skilled in the art that a sufficient or significant delay may actually include prevention, since the individual does not develop the disease. For example, it may delay advanced cancer (e.g., the development of metastasis).

As used herein, the "prevention" includes providing prevention against the occurrence or recurrence of a disease in an individual who may be predisposed to the disease but has not yet been diagnosed with the disease.

As used herein, an "inhibitory" function or activity is a decrease in function or activity as compared with the same condition other than the condition or parameter of interest, or alternatively as compared with another condition. For example, antibodies that inhibit tumor growth reduce the rate of tumor growth as compared with the rate of tumor growth in the absence of the antibodies.

The terms "subject", "individual", and "patient" are used interchangeably herein to refer to a mammal, including, but not limited to, human, bovine, horse, feline, canine, rodent, or primate. In some embodiments, the individual is a human.

An "effective amount" of an agent refers to an amount effective, at doses and for periods of time necessary, to achieve the desired therapeutic or prophylactic result. The specific dose may vary depending on one or more of: the selected particular agent, the dosage regimen to be followed, whether to be administered in combination with other compounds, the time of administration, the tissue to be imaged, and the physical delivery system that carries it.

The "therapeutically effective amount" of a substance/molecule, agonist, or antagonist of the present application may vary depending on factors such as the disease state, age, sex, and body weight of the individual, and the ability of the substance/molecule, agonist, or antagonist to elicit the desired response in the individual. A therapeutically effective amount is also an amount in which any toxic or detrimental effects of the substance/molecule, agonist, or antagonist are outweighed by the therapeutically beneficial effects. A therapeutically effective amount may be delivered in one or more administrations.

A "prophylactically effective amount" refers to an amount effective, at doses and for periods of time necessary, to achieve the desired prophylactic result. Typically, but not necessarily, since a prophylactic dose is used in a subject prior to or at an earlier stage of a disease, the prophylactically effective amount will be less than the therapeutically effective amount.

The terms "pharmaceutical formulation" and "pharmaceutical composition" refer to a preparation in a form that allows the biological activity of one or more active ingredients to be effective and does not comprise other components that are unacceptably toxic to the individual to which the formulation is to be administered. Such formulations may be sterile.

A "pharmaceutically acceptable carrier" refers to a non-toxic solid, semi-solid, or liquid filler, diluent, encapsulating material, formulation auxiliary, or carrier conventional in the art for use with a therapeutic agent, both of which together constitute a "pharmaceutical composition" for administration to an individual. The pharmaceutically acceptable carrier is non-toxic to the recipient at the dose and concentration employed and is compatible with the other ingredients of the formulation. A pharmaceutically acceptable carrier is suitable for the formulation employed.

It should be understood that where a chemical name is inconsistent with the chemical structure in the present application, if any, the chemical structure prevails.

It should be understood that embodiments of the present application described herein include "consisting" and/or "consisting essentially of' embodiments.

Reference to "about" a value or parameter herein includes (and describes) variations that are directed to that value or parameter per se. For example, description referring to "about X" includes description of "X".

As used herein, reference to "not" being a value or parameter generally means and describes a "different" value or parameter. For example, where a method is not used for treating type X cancer, it means that the method is used for treating cancers other than type X.

As used herein, the term "about X-Y" has the same meaning as "about X to about Y".

As used herein, modifying the amount of an ingredient or reactant in the present invention with the term "about" is a variation in the amount of a numerical value that may occur, for example, in typical measurements and liquid treatment procedures used for preparing concentrates or actually used solutions; in occasional errors in these procedures; in differences in manufacturing, sources, or the purity of ingredients used for preparing the composition or carrying out the method; etc. The term "about" also includes amounts that differ due to different equilibrium conditions relative to a composition resulting from a particular starting mixture. Whether or not modified by the term "about", equivalents of amounts are included in the claims. In one embodiment, the term "about" means being within 10% of the reported numerical value, preferably within 5% of the reported numerical value.

It will be understood by those skilled in the art that when a numerical value is recited in the claims, whether or not it is prefixed with "about", the actual value of the numerical value may vary by 10% (±10%) from the recited value, preferably by 5% (±5%).

As used herein and in the appended claims, the singular forms "a", "an", "or", and "the" include plural referents unless the context clearly dictates otherwise.

### 2. Antibody-drug conjugate

An anti-PD-L1 antibody can be conjugated to a cytotoxic or cytostatic moiety (including a pharmaceutically compatible salt thereof) to form an antibody-drug conjugate (ADC). Particularly suitable moieties for conjugation to the antibody are cytotoxic agents (e.g., chemotherapeutic agents), prodrug converting enzymes, radioisotopes or compounds or toxins (these moieties are collectively referred to as therapeutic agents). For example, the anti-PD-L1 antibody can be conjugated to a cytotoxic agent such as a chemotherapeutic agent or a toxin (e.g., a cytostatic agent or a cell killer, such as, for example, abrin, ricin A, Pseudomonas exotoxin, or diphtheria toxin).

The anti-PD-L1 antibody can be conjugated to a prodrug converting enzyme. The prodrug converting enzyme can be recombinantly fused with or chemically conjugated to the antibody by using a known method. Exemplary prodrug converting enzymes are carboxypeptidase G2, β-glucuronidase, penicillin-V-amidase, penicillin-G-amidase, β-lactamase, β-glucosidase, nitroreductase, and carboxypeptidase A.

Techniques for conjugating therapeutic agents to proteins, particularly to antibodies, are well known. (See, for example, Arnon et al., "Monoclonal Antibodies For Immunotargeting Of Drugs In Cancer Therapy", in Monoclonal Antibodies And Cancer Therapy (Reisfeld et al. eds., Alan R. Liss, Inc., 1985); Hellstrom et al., "Antibodies For Drug Delivery", in Controlled DrugDelivery (Robinson et al. eds., Marcel Dekker, Inc., 2nd edition, 1987); Thorpe, "AntibodyCarriers Of Cytotoxic Agents In Cancer Therapy: A Review", in Monoclonal Antibodies'84: Biological And Clinical Applications (Pinchera et al. eds., 1985); "Analysis, Results, and Future Prospective of the Therapeutic Use of Radiolabeled Antibody In Cancer Therapy", in Monoclonal Antibodies For CancerDetection And Therapy (Baldwin et al. eds., Academic Press, 1985); and Thorpe et al., 1982, Immunol. Rev. 62:119-58. See also, for example, PCT Publication No. WO 89/12624.)

The therapeutic agent can be conjugated in a manner that reduces the activity thereof unless it is cleaved from the antibody (e.g., by hydrolysis, by antibody degradation, or by a cleaving agent). Such a therapeutic agent is attached to the antibody by using a cleavable linker that is sensitive to cleavage in the intracellular environment or the tumor microenvironment of PD-L1-expressing cancer cells such that it is cleaved from the antibody when the conjugate is in that environment (e.g., in endosomes or for example due to pH sensitivity or protease sensitivity in lysosomal environments, in caveolae environments or in tumor microenvironment).

Typically, the ADC comprises a linker region between the therapeutic agent and the anti-PD-L1 antibody. As described above, typically, the linker is cleavable under intracellular conditions or in the tumor microenvironment such that the cleavage of the linker occurs in the intracellular environment (e.g., in lysosomes or endosomes or caveolae) or in the tumor microenvironment, leading to release of the therapeutic agent from the antibody. The linker can be, for example, a peptidyl linker that is cleaved by intracellular peptidases or proteases, including lysosomal or endosomal proteases. Typically, the peptidyl linker has a length of at least two amino acids or at least three amino acids. Cleaving agents may include cathepsins B and D, plasmin, etc. (see, e.g., Dubowchik and Walker, 1999, Pharm. Therapeutics 83: 67-123). The most typical peptidyl linkers are peptidyl linkers cleavable by enzymes present in PD-L1-expressing cells or within the tumor microenvironment. For example, peptidyl linkers cleavable by thiol-dependent protease cathepsin-B, which can be highly expressed in cancer tissues, can be used (e.g., a linker containing a Phe-Leu or Gly-Phe-Leu-Gly peptide). Other such linkers are described, for example, in U.S. Patent No. 6,214,345. In a specific embodiment, a peptidyl linker cleavable by an intracellular protease includes a Val-Cit linker or a Phe-Lys dipeptide (see, e.g., U.S. Patent 6,214,345, which describes the synthesis of doxorubicin using the Val-Cit linker). One advantage of using intracellular proteolysis to release the therapeutic agent is that the agent typically decays during conjugation and the serum stability of the conjugate is generally high.

The cleavable linker can be pH-sensitive, i.e., sensitive to hydrolysis at certain pH values. Typically, pH-sensitive linkers are hydrolyzable under acidic conditions. For example, acid-labile linkers that are hydrolyzable in lysosomes (e.g., hydrazones, hemicarbazones, thiosemicarbazones, cis-aconitic amide, orthoesters, acetals, and ketals) can be used. (See, e.g., U.S. Patent Nos. 5,122,368; 5,824,805; and 5,622,929; Dubowchik and Walker, 1999, Pharm. Therapeutics 83:67-123; and Neville et al., 1989, Biol. Chem. 264:14653-14661). Such linkers are relatively stable under neutral pH conditions, such as those in blood, but are not stable below pH 5.5 or 5.0 (the approximate pH of lysosomes). In certain embodiments, a hydrolyzable linker is a thioether linker (such as a thioether attached to a therapeutic agent via an acylhydrazone bond) (see, e.g., U.S. Patent No. 5,622,929).

Other linkers, such as disulfide linkers, are cleavable under reducing conditions. Disulfide linkers include those that can be formed with SATA (N-succinimidyl-S-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-α-methyl-α-(2-pyridyldithio)toluene), SPDB and SMPT. See, e.g., Thorpe et al., 1987, Cancer Res. 47:5924-5931; Wawrzynczak et al., Immunoconjugates: Antibody Conjugates in Radioimagery and Therapy of Cancer (C. W. Vogel eds, Oxford U. Press, 1987. See also U.S. Patent No. 4,880,935.)

The linker may also be a malonate linker (Johnson et al., 1995, Anticancer Res. 15: 1387-93), a maleimidobenzoyl linker (Lau et al., 1995, Bioorg-Med-Chem. 3 (10):1299-1304), or 3'-N-amide analogs (Lau et al., 1995, Bioorg-Med-Chem. 3 (10):1305-12). The linker may also be a malonate linker (Johnson et al., 1995, Anticancer Res. 15: 1387-93), a maleimidobenzoyl linker (Lau et al., 1995, Bioorg-Med-Chem. 3 (10):1299-1304), or 3'-N-amide analogs (Lau et al., 1995, Bioorg-Med-Chem. 3 (10):1305-12).

The linker may also be a non-cleavable linker, such as a maleimido-alkylene- or maleimido-aryl linker directly attached to a therapeutic agent (e.g., a drug). The active drug-linker is released by the degradation of the antibody.

Typically, the linker is substantially insensitive to the circulatory system environment, which means that when the ADC is present in plasma, no more than about 20%, typically no more than about 15%, more typically no more than about 10%, even more typically no more than about 5%, no more than about 3%, or no more than about 1% of the linker in an ADC sample is cleaved.

Whether the linker is substantially insensitive to the circulatory system environment can be determined, for example, by: incubating both (a) ADC ("ADC sample") and (b) an equimolar amount of an unconjugated antibody or therapeutic agent ("control sample") independently with plasma for a predetermined period of time (e.g., 2, 4, 8, 16, or 24 hours), and then comparing the amount of the unconjugated antibody or therapeutic agent present in the ADC sample as measured, for example, by high performance liquid chromatography, with the amount present in the control sample.

The anti-PDL1 antibody can be conjugated to the linker via a heteroatom in the antibody. Such heteroatoms can be present in their natural state on the antibody, or can be introduced into the antibody. In some aspects, the anti-PDL1 antibody will be conjugated to the linker via the nitrogen atom of the lysine residue. In other aspects, the anti-PDL1 antibody will be conjugated to the linker via the sulfur atom of the cysteine residue. The cysteine residue can be a residue that is naturally occurring or engineered into the antibody. Methods for conjugating linkers and drug-linkers to antibodies via lysine and cysteine residues are known in the art.

Exemplary antibody-drug conjugates also include camptothecin-based antibody-drug conjugates (i.e., the drug component is a camptothecin drug). Camptothecin is a topoisomerase inhibitor that has been proved to have anti-cancer activity. Typically, the camptothecin-based antibody-drug conjugate comprises a linker between the camptothecin drug and the anti-PDL1 antibody. The linker may be, for example, a cleavable linker (e.g., a peptidyl linker or a carbohydrate linker) or a non-cleavable linker (e.g., a linker released by the degradation of the antibody). The synthesis and structure of exemplary camptothecin drug linkers are described in PCT/US19/025968, which is incorporated herein for all purposes by reference in its entirety.

Other exemplary antibody-drug conjugates include maytansinoid antibody-drug conjugates (i.e., the drug component is a maytansinoid drug) and benzodiazepine antibody-drug conjugates (i.e., the drug component is a benzodiazepine (e.g., a pyrrolo[1,4]benzodiazepine dimer (PBD dimer), an indolobenzodiazepine dimer, and an oxazolidinebenzodiazepine dimer)).

Useful classes of cytotoxic agents conjugated to anti-PDL1 antibodies include, for example, anti-tubulin agents, DNA minor groove binders, DNA replication inhibitors, chemotherapeutic sensitizers, etc. Other exemplary classes of cytotoxic agents include anthracyclines, auristatins, camptothecins, duocarmycin, etoposide, maytansinoids, and vinca alkaloids. Some exemplary cytotoxic agents include camptothecins (e.g., irinotecan and DXD), auristatins (e.g., auristatin T, auristatin E, AFP, monomethylauristatin F (MMAF), lipophilic monomethylauristatin F, monomethylauristatin E (MMAE)), DNA minor groove binders (e.g., enediynes and lexitropsins), duocarmycin, taxanes (e.g., taxol and docetaxel), vinca alkaloids, nicotinamide phosphoribosyltransferase inhibitors (NAMPTi), Tubulysin M, doxorubicin, morpholino-doxorubicin, and cyclomorpholino-doxorubicin.

The cytotoxic agent may be a chemotherapeutic agent such as, for example, doxorubicin, taxol, melphalan, vinca alkaloids, methotrexate, mitomycin C, or etoposide. The agent may also be an analog of CC-1065, calicheamicin, maytansine, a dolastatin 10 analog, rhizoxin, or palytoxin.

The cytotoxic agent may also be auristatin. The auristatin can be an auristatin E derivative, such as an ester formed between auristatin E and a keto acid. For example, auristatin E can react with p-acetylbenzoic acid or benzoylvaleric acid to produce AEB and AEVB, respectively. Other typical auristatins include auristatin T, AFP, MMAF, and MMAE. The synthesis and structure of various auristatins are described, for example, in US 2005-0238649 and US 2006-0074008.

The present invention discloses an antibody-drug conjugate, a preparation method therefor, and the use thereof, in particular a conjugate of an anti-PD-L1 antibody and a novel topoisomerase (TOP) I inhibitor that is more tumor-sensitive, a pharmaceutical composition thereof, a preparation method therefor, and the use thereof. The resulting antibody-drug conjugate has been found to have better activity, such as potent anti-tumor activity, by anti-tumor experiments. It significantly improves the survival of tumor-bearing animals, has significantly reduced toxicity, less burden on the body of the experimental animals, and greatly reduced minimum effective dose of the small molecule drug when used alone, expands the therapeutic window thereof, is promising for use in the development of drugs for treating a variety of diseases such as tumors, and shows good application prospects and value.

The anti-PDL1 antibody-drug conjugate of the present invention is a novel PDL1-targeted antibody-drug conjugate (ADC) consisting essentially of three moieties, namely a monoclonal antibody targeting PDL1, a small molecule drug (payload), and a tumor microenvironment-sensitive linker (Linker), wherein the loaded drug is a novel topoisomerase (TOP) I inhibitor that is more tumor-sensitive. After the anti-PDL1 antibody-drug conjugate of the present invention enters the body, one part of the anti-PDL1 antibody-drug conjugate can release the loaded drug in the tumor microenvironment upon the binding of the antibody moiety thereof specifically to highly expressed PDL1 on tumor cells while the other part enters the cancer cells via PDL1-mediated drug internalization. In a special environment within the tumors, the peptide linker of the anti-PDL1 antibody-drug conjugate of the present invention is cleaved to efficiently release the payload, and the loaded small molecule drug exerts a cytotoxic effect and can thus kill the cancer cells in a targeted manner. Due to the relatively high lipophilicity, some payloads can exert a "bystander effect", enabling their action after entering cells surrounding cancer cells, while PDL1-ADCs have the potential to block PD-1/PDL1 immune checkpoints. These anti-cancer mechanisms of action are also effective against low-antigen-expressing and negative tumor cells. The anti-PDL1 antibody-drug conjugate of the present invention is expected to be an ideal therapeutic drug for the following patients: patients who have shown an unsatisfactory response to PD-1/PDL1 inhibitor therapy, or progression following PD-1/PDL1 inhibitor therapy.

The anti-PDL1 antibody-drug conjugate of the present invention has the following characteristics:
(1) A new generation of linker type that is relatively sensitive to the tumor microenvironment is used to ensure the stability of ADC drugs in the blood circulation and enable efficient release of the loaded small molecule drug into tumor cells or when in the tumor microenvironment.
(2) Loaded drugs (Payloads) with the new generation of mechanism of action are used to efficiently kill cancer cells with different levels of PDL1 expression.
(3) A new generation of antibody conjugation technology is used to ensure the consistency and accuracy of the antibody conjugation site, so that the half-life and immunogenicity thereof are similar to that of the antibody, while the PD-1/PDL1 immune checkpoint blocking function of the antibody moiety is retained.

In some aspects, the present invention provides an antibody-drug conjugate, a prodrug, pharmaceutically acceptable salt, or solvate thereof, or a solvate of the pharmaceutically acceptable salt, wherein the antibody-drug conjugate has a structure represented by formula (I):

TP-[L1-L2-L3-D]ₖ (I)

wherein
TP is a targeting moiety selectively binding or recognizing PDL1;
L1 is an extension unit connecting TP and L2, wherein TP is connected to L1 via an active group (e.g., sulfhydryl or amino group), preferably wherein L1 is conjugated to an exposed interchain cysteine sulfhydryl group (-SH) in TP and/or a cysteine sulfhydryl group (-SH) resulting from site-directed mutagenesis;
L2 is an optional amino acid residue or a short peptide consisting of 2-10 amino acid residues;
L3 is a spacer element;
D is a biologically active molecule; and
k represents any numerical value between 0.1 and 10.0.

With reference to the antibody-drug conjugate, the subscript k represents the drug loading and, depending on the context, may represent the molecular number of the drug-linker molecules attached to the individual antibody molecule; therefore, it is an integer value; alternatively, k can represent the average drug loading and therefore can be an integer or non-integer value. The average drug loading represents the average number of drug-linker molecules per antibody in a population. Usually, but not always, when referring to antibodies, such as monoclonal antibodies, we refer to a population of antibody molecules. In a composition comprising a population of antibody-drug conjugate molecules, the average drug loading is an important mass attribute, as it determines the amount of the drug that can be delivered to the target cell. The percentage of unconjugated antibody molecules in the composition is included in the average drug loading value.

In a preferred aspect of the present invention, when referring to a composition comprising a population of antibody-drug conjugate compounds, the average drug loading is from 1 to about 16, preferably about 2 to about 14, more preferably from about 2 to about 10.

For MMAE and camptothecin ADCs, such as those exemplified herein, a preferred average drug loading is about 2, 4, or 8, and a particularly preferred average drug loading is about 8. In one embodiment, a preferred average drug loading for an MMAE ADC is 2 or 4. In one embodiment, a preferred average drug loading for a camptothecin ADC is 4 or 8. In an exemplary embodiment, the drug-linker is conjugated to a cysteine residue at a reduced interchain disulfide. In some aspects, the actual drug loading per individual antibody molecule in a population of antibody-drug conjugate compounds is from 1 to 10 (or from 6 to 10 or from 6 to 8), with the predominant drug loading being 8.

In some embodiments of formula (I), the TP is an antibody or an antigen-binding fragment thereof.

In some embodiments of formula (I), the antigen-binding fragment is selected from a full-length immunoglobulin, a single-chain Fv (scFv) fragment, an Fab fragment, an Fab' fragment, F(ab')₂, an Fv fragment, a disulfide-stabilized Fv fragment (dsFv), (dsFv)₂, an Fv-Fc fusion, an scFv-Fc fusion, an scFv-Fv fusion, a diabody, a tribody, a tetrabody, or any combination thereof.

In some embodiments of formula (I), the TP is a humanized antibody or an antigen-binding fragment thereof.

In some embodiments of formula (I), the TP comprises a heavy chain variable region (VH) and a light chain variable region (VL), wherein the heavy chain variable region (VH) comprises heavy chain complementarity determining region 1 (HCDR1), HCDR2, and HCDR3 contained in SEQ ID NO: 7, and the light chain variable region (VL) comprises light chain complementarity determining region 1 (LCDR1), LCDR2, and LCDR3 contained in SEQ ID NO: 8.

In some embodiments of formula (I), the amino acid sequence of HCDR1 is SEQ ID NO: 1, the amino acid sequence of HCDR2 is SEQ ID NO: 2, and the amino acid sequence of HCDR3 is SEQ ID NO: 3; and the amino acid sequence of LCDR1 is SEQ ID NO: 4, the amino acid sequence of LCDR2 is SEQ ID NO: 5, and the amino acid sequence of LCDR3 is SEQ ID NO: 6.

In some embodiments of formula (I), the TP comprises an Fc sequence of human IgG.

In some embodiments of formula (I), the IgG is selected from IgG1, IgG2, IgG3, and IgG4.

In some embodiments of formula (I), the TP comprises a heavy chain variable region (VH), and the amino acid sequence of the heavy chain variable region (VH) has at least 70%, at least 80%, at least 90%, at least 95%, at least 99%, or 100% identity to SEQ ID NO: 7.

In some embodiments of formula (I), the TP comprises a light chain variable region (VL), and the amino acid sequence of the light chain variable region (VL) has at least 70%, at least 80%, at least 90%, at least 95%, at least 99%, or 100% identity to SEQ ID NO: 8.

In some embodiments of formula (I), the TP comprises a heavy chain (HC), and the amino acid sequence of the heavy chain (HC) has at least 70%, at least 80%, at least 90%, at least 95%, at least 99%, or 100% identity to SEQ ID NO: 9.

In some embodiments of formula (I), the TP comprises a light chain (LC), and the amino acid sequence of the light chain (LC) has at least 70%, at least 80%, at least 90%, at least 95%, at least 99%, or 100% identity to SEQ ID NO: 10.

In some embodiments of formula (I), the TP is a multispecific antibody.

In some embodiments of formula (I), the TP is an afucosylated antibody.

In some embodiments of formula (I), the Fc region comprises a C-terminal lysine.

In some embodiments of formula (I), the Fc region comprises a deletion of the C-terminal lysine.

In some embodiments of formula (I), the L1 is selected from 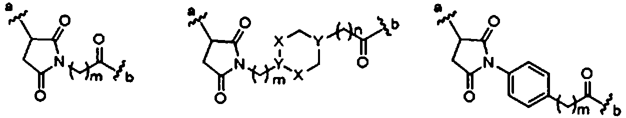 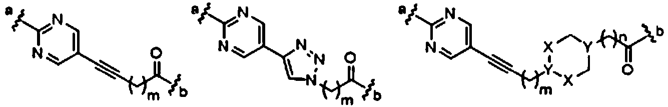 wherein L1 is connected to TP at position a via an S atom and to L2 at position b, m is any integer between 1 and 10, n is any integer between 1 and 10, and X and Y are each independently C, O, S, or N.

In some embodiments of formula (I), the L1 is 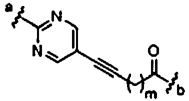
wherein m is selected from 1, 2, 3, 4, or 5,
wherein L1 is connected to TP at position a via an S atom and to L2 at position b.

In some embodiments of formula (I), the L1 is 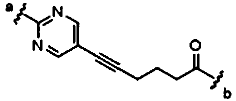 wherein L1 is connected to TP at position a via an S atom and to L2 at position b.

In some embodiments, L1 is connected to a cysteine of TP (more specifically, the sulfhydryl group of the cysteine) at position a.

In some embodiments of formula (I), the L2 is selected from 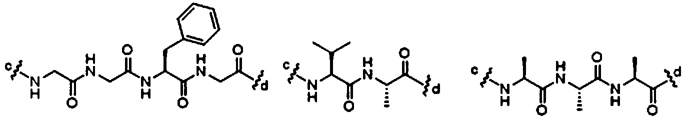 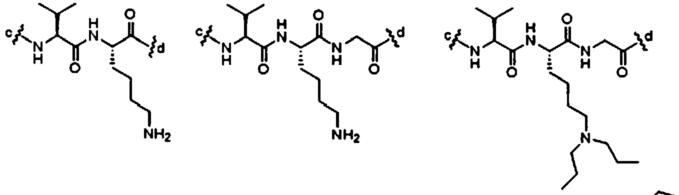 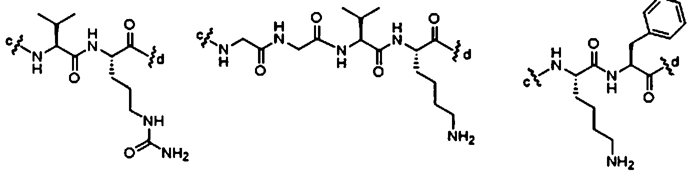 wherein L2 is connected to L1 at position c and to L3 at position d.

In some embodiments of formula (I), the L2 is 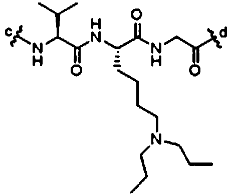 wherein L2 is connected to L1 at position c and to L3 at position d.

In some embodiments of formula (I), the L3 is a single bond or a group selected from: 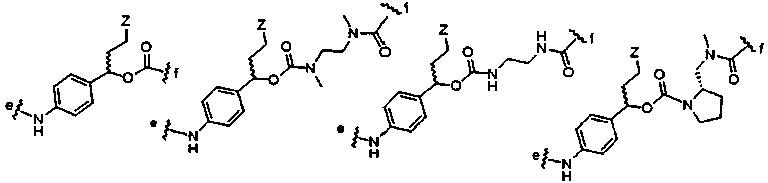
wherein L3 is connected to L2 at position e and to D at position f,
Z is selected from hydrogen, halogen, C₁-C₂₀ alkyl, C₁-C₂₀ haloalkyl, cyano, sulfo, carboxyl, C₁-C₂₀ alkoxy, C₂-C₁₀ unsaturated alkyl, a natural amino acid, an unnatural amino acid, and a short peptide consisting of the above amino acids.

In some embodiments of formula (I), Z is selected from 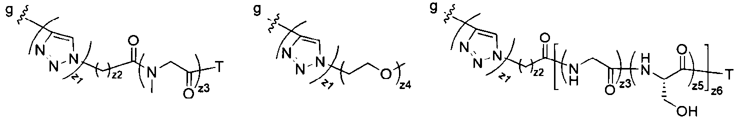 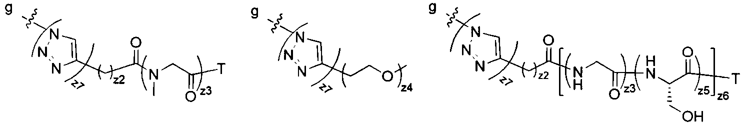 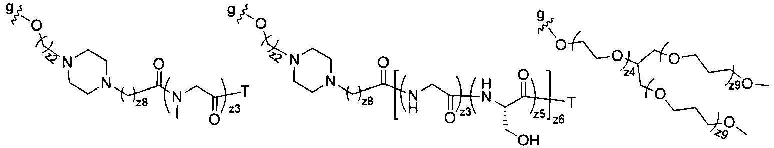 wherein Z is connected to the remainder of L3 at position g, zl-z9 are each independently an integer between 0 and 10, and T is NH₂ or OH.

In some embodiments of formula (I), D is a cytotoxic agent.

In some embodiments of formula (I), D is selected from 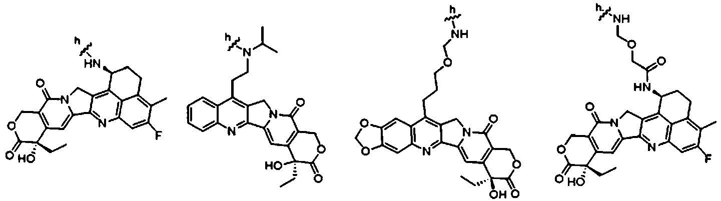 wherein D is connected to L3 at position h.

In some embodiments of formula (I), the antibody-drug conjugate is selected from:

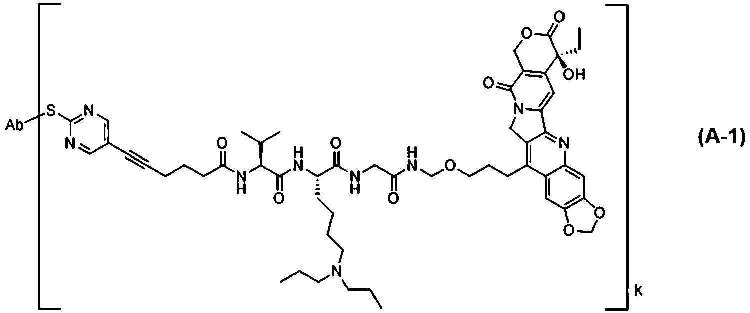

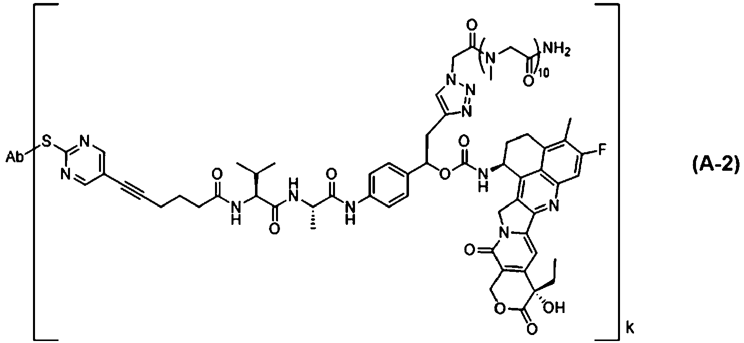

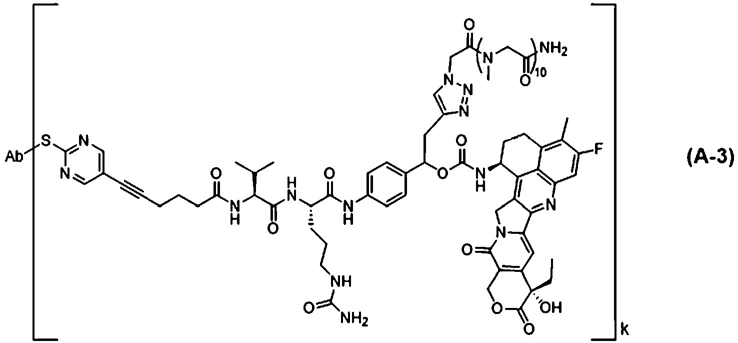

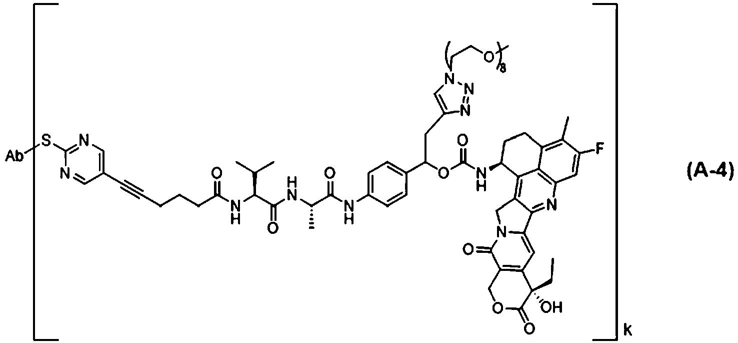

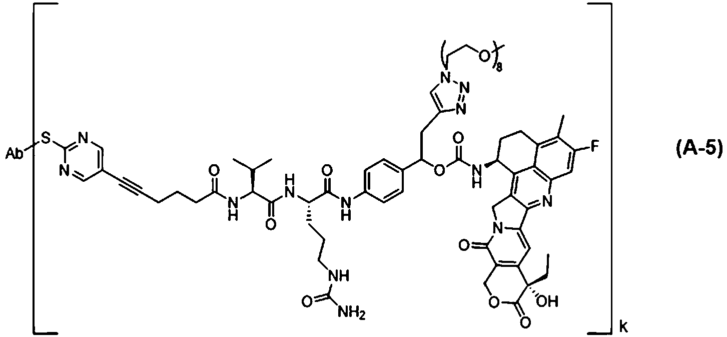

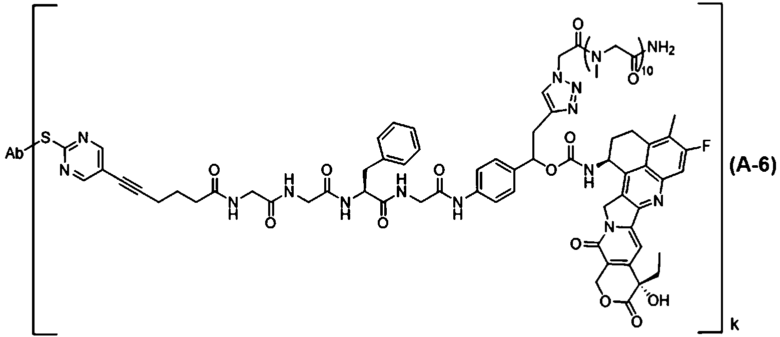

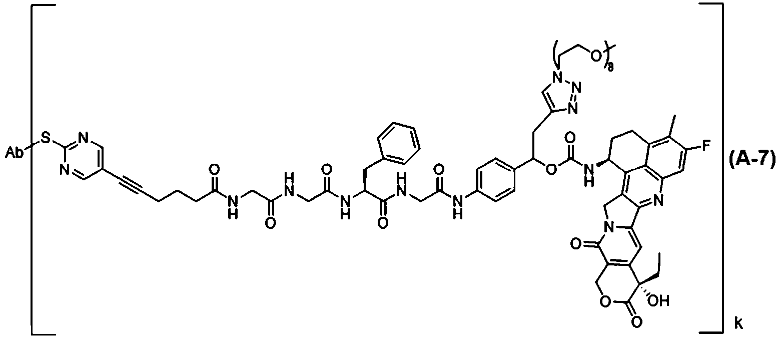

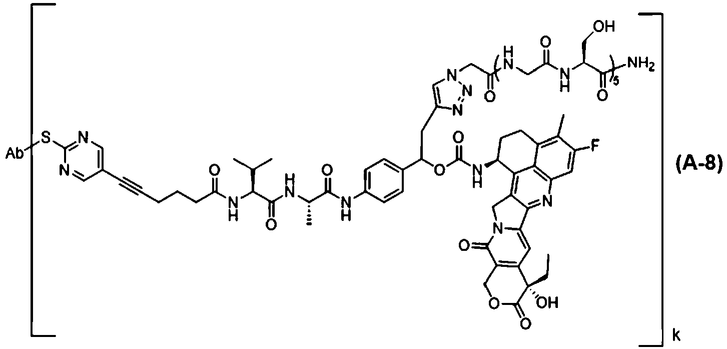

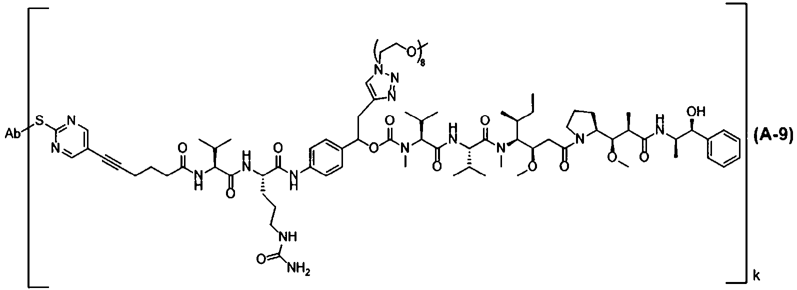

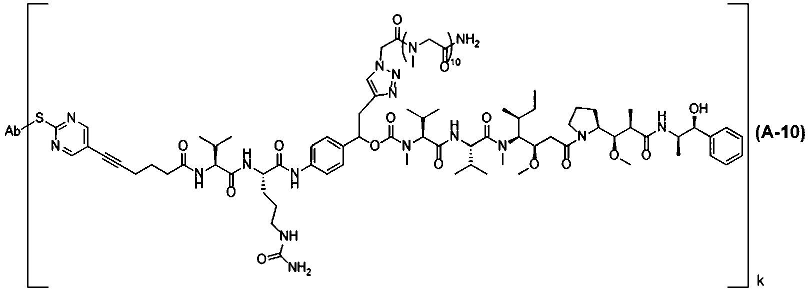

Ab represents the antibody or antigen-binding fragment thereof as defined in the previous embodiments.

In some embodiments of formula (I), k is 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6. 1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, or in a range between any two thereof. In further embodiments, k is 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6. 1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, or in a range between any two thereof. In further embodiments, k is 4.0, 6.0, or 8.0.

### 3. Method of use

The anti-PDL1 antibody-drug conjugate of the present invention can be used for treating cancer. Some of such cancers show detectable PDL1 levels measured at the protein (e.g., by immunoassay using one of the exemplified antibodies) or mRNA level. Some of such cancers show elevated PDL1 levels relative to the same type of non-cancerous tissue, preferably from the same patient. An exemplary level of PDL1 on treatable cancer cells is 5,000-500,000 PDL1 molecules per cell; however, those with higher or lower levels can be treated. Optionally, the level of PDL1 in cancer is measured prior to treatment.

Examples of cancers related to PDL1 expression and suitable for treatment include melanoma, non-small cell lung cancer (NSCLC), small cell lung cancer (SCLC), head and neck cancer, breast cancer (e.g., triple negative breast cancer (TNBC)), pancreatic cancer, ovarian cancer, urothelial cancer, hepatocellular carcinoma (HCC), esophageal cancer, bladder cancer, gastric cancer, colorectal cancer, cervical cancer, kidney cancer, prostate cancer, pancreatic cancer, and glioma. In some embodiments, the antibody or antibody-drug conjugate of the present invention is used for treating melanoma. In some embodiments, the antibody or antibody-drug conjugate of the present invention is used for treating NSCLC. In some embodiments, the antibody or antibody-drug conjugate of the present invention is used for treating SCLC. In some embodiments, the antibody or antibody-drug conjugate of the present invention is used for treating head and neck cancer. In some embodiments, the antibody or antibody-drug conjugate of the present invention is used for treating breast cancer. In some embodiments, the antibody or antibody-drug conjugate of the present invention is used for treating TNBC. Triple negative breast cancer is a term specific for cancer that lacks detectable estrogen and progestin receptors and lacks HER2/neu overexpression. In some embodiments, the antibody or antibody-drug conjugate of the present invention is used for treating pancreatic cancer. In some embodiments, the antibody or antibody-drug conjugate of the present invention is used for treating ovarian cancer. In some embodiments, the antibody or antibody-drug conjugate of the present invention is used for treating urothelial cancer. In some embodiments, the antibody or antibody-drug conjugate of the present invention is used for treating HCC. In some embodiments, the antibody or antibody-drug conjugate of the present invention is used for treating esophageal cancer. In some embodiments, the antibody or antibody-drug conjugate of the present invention is used for treating gastric cancer. In some embodiments, the antibody or antibody-drug conjugate of the present invention is used for treating cervical cancer. In some embodiments, the antibody or antibody-drug conjugate of the present invention is used for treating kidney cancer. In some embodiments, the antibody or antibody-drug conjugate of the present invention is used for treating prostate cancer. In some embodiments, the antibody or antibody-drug conjugate of the present invention is used for treating pancreatic cancer. In some embodiments, the antibody or antibody-drug conjugate of the present invention is used for treating glioma. The treatment can be applied to patients with these types of primary or metastatic tumors. The treatment can also be applied to patients refractory to conventional treatment or who have relapsed after responding to such treatment.

The antibody-drug conjugate of the present invention is administered in an effective regimen that means a dose, route of administration, and frequency of administration which can delay onset, reduce severity, inhibit further exacerbation, and/or ameliorate at least one sign or symptom of cancer. If the patient had already had cancer, the regimen may be referred to as a therapeutically effective regimen. If the patient is at increased risk of developing cancer relative to the general population, but has not experienced symptoms, the regimen may be referred to as a prophylactically effective regimen. In some cases, therapeutic or prophylactic efficacy can be observed in individual patients relative to historical controls or past experience in the same patient. In other cases, therapeutic or prophylactic efficacy in preclinical or clinical trials can be demonstrated relative to a control population of untreated patients.

An exemplary dose of the antibody-drug conjugate may be 1 µg/kg to 15 mg/kg (e.g., 0.1 mg/kg to 20 mg/kg), e.g., 1 mg/kg to 7.5 mg/kg, or 2 mg/kg to 7.5 mg/kg, or 3 mg/kg to 7.5 mg/kg of the body weight of the subject, or 0.1-20 mg/kg of the body weight, or 0.5-5 mg/kg of the body weight (e.g., 0.5 mg/kg, 1 mg/kg, 2 mg/kg, 3 mg/kg, 4 mg/kg, 5 mg/kg, 6 mg/kg, 7 mg/kg, 8 mg/kg, 9 mg/kg, or 10 mg/kg), or as a fixed dose of 10-1500 mg or 200-1500 mg. In some methods, the patient is administered a dose of at least 1 mg/kg, at least 1.5 mg/kg, at least 2 mg/kg, at least 3 mg/kg, at least 8 mg/kg, or at least 10 mg/kg, once every one week, once every three weeks, or less frequently. The dose depends on factors such as the frequency of administration, the condition of the patient and the response, if any, to the previous treatment, whether the treatment is prophylactic or therapeutic, and whether the disorder is acute or chronic.

The administration may be parenteral, intravenous, oral, subcutaneous, intraarterial, intracranial, intrathecal, intraperitoneal, local, intranasal, or intramuscular. The administration can also be directly localized to the tumor. It is preferably administered into the systemic circulation by intravenous or subcutaneous administration. Intravenous administration may be performed, for example, by infusion over a period of time (e.g., 30-90 min) or by a single bolus injection.

The frequency of administration depends on factors such as the half-life of the conjugate in circulation, the condition of the patient, and the route of administration. The frequency may be once a day, once a week, once a month, once a quarter, or at irregular intervals in response to changes in the condition in the patient or the progression of the cancer being treated. During a continuous course of treatment, an exemplary frequency of intravenous administration is between twice a week and once a quarter, but the administration may also be at a higher or lower frequency. During a continuous course of treatment, other exemplary frequencies of intravenous administration are between once a week or three times every four weeks, but the administration may also be at a higher or lower frequency. For subcutaneous administration, exemplary dosing frequencies are once a day to once a month, but the administration may also be at a higher or lower frequency.

The number of doses administered depends on the nature of the cancer (e.g., presenting acute or chronic symptoms) and the response of the disorder to the treatment. Between 1 and 10 doses are usually sufficient for acute disorders or acutely exacerbated chronic disorders. Sometimes, single bolus doses (optionally in separate forms) are sufficient for acute disorders or acutely exacerbated chronic disorders. Recurrence or acute exacerbation of acute disorders can be treated repeatedly. For chronic disorders, antibodies may be administered at regular intervals, e.g., once a week, once every two weeks, once a month, once a quarter, or once every six months for at least 1, 5, or 10 years or the lifespan of the patient.

Pharmaceutical compositions for parenteral administration are preferably sterile and substantially isotonic and are manufactured under GMP conditions. Pharmaceutical compositions may be provided in a unit dosage form (i.e., a dose for a single administration). Pharmaceutical compositions may be formulated using one or more physiologically acceptable carriers, diluents, excipients, or auxiliaries. The formulation depends on the chosen route of administration. For injection, the antibody can be formulated in an aqueous solution, preferably in a physiologically compatible buffer such as Hank's solution, Ringer's solution, or normal saline, or acetate buffer (to reduce discomfort at the injection site). The solution may contain formulation agents such as suspending agents, stabilizers and/or dispersing agents. Alternatively, before use, the antibody may be in a lyophilized form for constitution in a suitable vehicle (e.g., sterile pyrogen-free water). The concentration of the antibody in a liquid formulation may be, for example, 1-100 mg/ml, such as 10 mg/ml.

Treatment with the antibody-drug conjugate of the present invention may be combined with chemotherapy, radiation, stem cell therapy, surgery, or other therapies that are effective for the disorder being treated. Useful classes of other agents that can be administered with PDL1-targeted antibody-drug conjugates as described herein include, for example, antibodies targeting other receptors expressed on cancer cells, anti-tubulin agents (e.g., auristatins), DNA minor groove binders, DNA replication inhibitors, alkylating agents (e.g., platinum complexes such as cisplatin, mono(platinum), bis(platinum) and trinuclear platinum complexes, and carboplatin), anthracyclines, antibiotics, antifolates, antimetabolites, chemotherapeutic sensitizers, duocarmycin, etoposide, fluorinated pyrimidines, ionophores, lexitropsins, nitrosourea, platinols, pre-forming compounds, purine antimetabolites, puromycin, radiosensitizers, steroids, taxanes, topoisomerase inhibitors, vinca alkaloids, etc.

Treatment with an antibody-drug conjugate, optionally in combination with any of the other agents or regimens described above, can increase the median progression-free survival or overall survival time of a patient with a tumor (e.g., melanoma, non-small cell lung cancer (NSCLC), small cell lung cancer (SCLC), head and neck cancer, breast cancer (e.g., triple negative breast cancer (TNBC)), pancreatic cancer, ovarian cancer, urothelial cancer, hepatocellular carcinoma (HCC), esophageal cancer, gastric cancer, colorectal cancer, and cervical cancer), especially in the case of recurrent or refractory tumors, by at least 30% or 40%, but preferably by 50%, 60% to 70%, or even 100% or longer, as compared with the same treatment (e.g., chemotherapy) without the antibody-drug conjugate. Additionally or alternatively, a treatment comprising the antibody-drug conjugate (e.g., standard chemotherapy) can increase the complete response rate, partial response rate, or objective response rate (complete + partial) of a patient with a tumor by at least 30% or 40%, but preferably by 50%, 60% to 70%, or even 100%, as compared with the same treatment (e.g., chemotherapy) without the antibody-drug conjugate.

### 4. Pharmaceutical formulation

Although the antibody-drug conjugate described herein may be used alone (e.g., by administration), it is generally preferably present in a composition or formulation.

In one aspect, the composition is a pharmaceutical composition (e.g., formulation, preparation, or agent) comprising the antibody-drug conjugate described herein and a pharmaceutically acceptable carrier, diluent, or excipient.

In one aspect, the composition is a pharmaceutical composition comprising at least one of the antibody-drug conjugates described herein and one or more other pharmaceutically acceptable ingredients well known to those skilled in the art, including but not limited to pharmaceutically acceptable carriers, diluents, excipients, adjuvants, fillers, buffers, preservatives, antioxidants, lubricants, stabilizers, solubilizers, surfactants (e.g., wetting agents), masking agents, colorants, flavorings, and sweeteners.

In one aspect, the composition further comprises other active agents, such as other therapeutic or prophylactic agents.

Suitable carriers, diluents, excipients, etc. can be found in standard pharmaceutical literatures. See, for example, Handbook of Pharmaceutical Additives, 2nd edition (M. Ash and I. Ash eds.), 2001 (Synapse Information Resources, Inc., Endicott, New York, U.S., Remington's Pharmaceutical Sciences, 20th edition, Lippincott, Williams & Wilkins, 2000; and Handbook of Pharmaceutical Excipients, 2nd edition, 1994.

Another aspect of the present invention relates to a method for preparing a pharmaceutical composition, which comprises mixing at least one [11C]-radiolabeled antibody-drug conjugate or antibody-drug conjugate-like compound as defined herein with one or more other pharmaceutically acceptable ingredients (e.g., carriers, diluents, and excipients) well known to those skilled in the art. If formulated as discrete units (e.g., tablets), each unit contains a predetermined amount (dose) of the active compound.

As used herein, the term "pharmaceutically acceptable" relates to compounds, ingredients, materials, compositions, dosage forms, etc., which are, within the scope of sound medical judgment, suitable for contact with the tissue of the subject in question (e.g., a human), without undue toxicity, irritation, allergic reactions, or other problems or complications, which is commensurate with a rational benefit/risk ratio. Each carrier, diluent, excipient, etc. must also be "acceptable" in the sense of being compatible with the other ingredients of the formulation.

Formulations can be prepared by any method well known in the pharmaceutical field. Such methods include the step of associating the active compound with a carrier that constitutes one or more auxiliary ingredients. Generally, formulations are prepared by uniformly and intimately associating the active compound with a carrier (e.g., a liquid carrier or a finely divided solid carrier) and then shaping the product as needed.

The formulation may be prepared to provide rapid or slow release; immediate, delayed, timed, or sustained release; or a combination thereof.

Formulations suitable for parenteral administration (e.g., by injection) include aqueous or non-aqueous, isotonic, pyrogen-free sterile liquids (e.g., solutions or suspensions), wherein the active ingredient is dissolved, suspended or otherwise provided (e.g., in liposomes or other microparticles). Such liquids may additionally contain other pharmaceutically acceptable ingredients, such as antioxidants, buffers, preservatives, stabilizers, bacteriostats, suspending agents, thickeners, and solutes, which can make the formulation isotonic with the blood (or other relevant bodily fluids) of the intended recipient. Examples of excipients include, for example, water, alcohols, polyols, glycerol, vegetable oils, etc. Examples of suitable isotonic carriers for such formulations include a sodium chloride injection, Ringer's solution, or lactated Ringer's injection. Typically, the concentration of the active ingredient in the liquid is from about 1 ng/ml to about 10 µg/ml, for example from about 10 ng/ml to about 1 µg/ml. The formulation may be present in unit dose or multi-dose sealed containers (e.g., ampoules and vials) and may be stored under freeze-drying (lyophilization) conditions, requiring only the addition of a sterile liquid carrier (e.g., water) for injection (immediately) before use. Temporary injection solutions and suspensions can be prepared from sterile powders, granules, and tablets.

### 5. Article of manufacture and kit

In another aspect, an article of manufacture or kit comprising an anti-PDL1 antibody-drug conjugate described herein is provided. The article of manufacture or kit may also comprise instructions for use of the anti-PDL1 antibody-drug conjugates described herein in the methods of the present invention. Therefore, in certain embodiments, the article of manufacture or kit comprises instructions for use of the anti-PDL1 antibody-drug conjugate described herein in a method for treating cancer (e.g., melanoma, non-small cell lung cancer (NSCLC), small cell lung cancer (SCLC), head and neck cancer, breast cancer (e.g., triple negative breast cancer (TNBC)), pancreatic cancer, ovarian cancer, urothelial cancer, hepatocellular carcinoma (HCC), esophageal cancer, bladder cancer, gastric cancer, colorectal cancer, cervical cancer, kidney cancer, prostate cancer, pancreatic cancer, and glioma), the method comprising administering to the subject an effective amount of the anti-PDL1 antibody-drug conjugate described herein. In some embodiments, the subject is a human.

The article of manufacture or kit can further comprise a container. Suitable containers include, for example, bottles, vials (e.g., dual-compartment vials), injectors (e.g., single-compartment or dual-compartment injectors), and test tubes. In some embodiments, the container is a vial. The container can be made of various materials, such as glass or plastic. The container accommodates the formulation.

The article of manufacture or kit may also comprise a label or package insert on or associated with the container that may indicate instructions for reconstitution and/or use of the formulation. The label or package insert may further indicate that the formulation is useful or intended for subcutaneous, intravenous (e.g., intravenous infusion), or other mode of administration to treat cancer (e.g., melanoma, non-small cell lung cancer (NSCLC), small cell lung cancer (SCLC), head and neck cancer, breast cancer (e.g., triple negative breast cancer (TNBC)), pancreatic cancer, ovarian cancer, urothelial cancer, hepatocellular carcinoma (HCC), esophageal cancer, bladder cancer, gastric cancer, colorectal cancer, cervical cancer, kidney cancer, prostate cancer, pancreatic cancer, and glioma) in a subject. The container accommodating the formulation can be a disposable vial or a multi-use vial, which allows repeated administration of the reconstituted formulation. The article of manufacture or kit may also comprise a second container containing a suitable diluent. The article of manufacture or kit may also comprise other materials desired from a commercial, therapeutic, and user perspective, including other buffers, diluents, filters, needles, syringes, and package inserts printed with instructions for use.

The article of manufacture or kit herein also optionally comprises a container containing a second agent, wherein the anti-PDL1 antibody-drug conjugate is a first agent, and the article of manufacture or kit further comprises instructions on a label or package insert for treating a subject with an effective amount of the second agent. In some embodiments, the second agent is used for eliminating or reducing the severity of one or more adverse events.

In some embodiments, the anti-PDL1 antibody-drug conjugate is present in the container as a lyophilized powder. In some embodiments, the lyophilized powder is in a hermetically sealed container such as a vial, ampoule, or pouch, wherein the container indicates the amount of the active agent. In the case of administration of the drug by injection, an ampoule of sterile water for injection or normal saline may, for example, optionally be provided as part of the kit so that the ingredients may be mixed prior to administration. Such kits may also comprise, if desired, one or more of various conventional drug components, such as, for example, a container having one or more pharmaceutically acceptable carriers, an additional container, etc. This is clear to those skilled in the art. The kit may also comprise printed instructions as inserts or labels indicating the amount of components to be administered, administration guidelines, and/or instructions for mixing the components.

**Sequence Listing**

| **SEQ ID NO** | **Gene Name** | **Amino acid sequence** |
|---|---|---|
| 1. | MAB20 heavy chain CDR1 | SYTMN |
| 2. | MAB20 heavy chain CDR2 | SISSGSDYLYYADSVKG |
| 3. | MAB20 heavy chain CDR3 | NELRWYPQAGAFDR |
| 4. | MAB20 light chain CDR1 | SGSSSYIESSYVG |
| 5. | MAB20 light chain CDR2 | DDDMRPS |
| 6. | MAB20 light chain CDR3 | EIWRSGLGGV |
| 7. | MAB20 heavy chain variable region | EVQLVQSGGGLVKPGGSLRLSCAASGFTFSSYTMNWVR QAPGKGLEWVSSISSGSDYLYYADSVKGRFTISRDNAKN SLYLQMNSLRAEDTAVYYCARNELRWYPQAGAFDRWG QGTMVTVSS QSVVTQPPSMSAAPGQRVTISCSGSSSYIESSYVGWYQQL PGTAPRLLIYDDDMRPSGIPDRFSGSKSGTSATLAITGLQT GDEADYYCEIWRSGLGGVFGGGTKLTVLSQP |
| 8. | MAB20 light chain | QSVVTQPPSMSAAPGQRVTISCSGSSSYIESSYVGWYQQL PGTAPRLLIYDDDMRPSGIPDRFSGSKSGTSATLAITGLQT GDEADYYCEIWRSGLGGVFGGGTKLTVLSQP |
| | variable region | |
| 9. | MAB20 heavy chain | EVQLVQSGGGLVKPGGSLRLSCAASGFTFSSYTMNWVR QAPGKGLEWVSSISSGSDYLYYADSVKGRFTISRDNAKN SLYLQMNSLRAEDTAVYYCARNELRWYPQAGAFDRWG QGTMVTVSSASTKGPSVFPLAPSSKSTSGGTAALGCLVK DYFPEPVTVSWNSGALTSGVHTFPAVLQSSGLYSLSSVVT VPSSSLGTQTYICNVNHKPSNTKVDKKVEPKSCDKTHTC PPCPAPELLGGPSVFLFPPKPKDTLMISRTPEVTCVVVDVS HEDPEVKFNWYVDGVEVHNAKTKPREEQYASTYRVVSV LTVLHQDWLNGKEYKCKVSNKALPAPIEKTISKAKGQPR EPQVYTLPPSREEMTKNQVSLTCLVKGFYPSDIAVEWES NGQPENNYKTTPPVLDSDGSFFLYSKLTVDKSRWQQGN VFSCSVMHEALHNHYTQKSLSLSPGK |
| 10. | MAB20 light chain | QSVVTQPPSMSAAPGQRVTISCSGSSSYIESSYVGWYQQL PGTAPRLLIYDDDMRPSGIPDRFSGSKSGTSATLAITGLQT GDEADYYCEIWRSGLGGVFGGGTKLTVLSQPKAAPSVTL FPPSSEELQANKATLVCLISDFYPGAVTVAWKADSSPVK AGVETTTPSKQSNNKYAASSYLSLTPEQWKSHKSYSCQV THEGSTVERTVALTEC |
| 11. | Human PDL1 ECD | FTVTVPKDLYVVEYGSNMTIECKFPVEKQLDLAALIVYW EMEDKNIIQFVHGEEDLKVQHSSYRQRARLLKDQLSLGN AALQITDVKLQDAGVYRCMISYGGADYKRITVKVNAPY NKINQRILVVDPVTSEHELTCQAEGYPKAEVIWTSSDHQ VLSGKTTTTNSKREEKLFNVTSTLRINTTTNEIFYCTFRRL DPEENHTAELVIPELPLAHPPNER |
| 12. | Monkey PDL1 ECD | FTVTVPKDLYVVEYGSNMTIECKFPVEKQLDLTSLIVYW EMEDKNIIQFVHGEEDLKVQHSNYRQRAQLLKDQLSLGN AALRITDVKLQDAGVYRCMISYGGADYKRITVKVNAPY NKINQRILVVDPVTSEHELTCQAEGYPKAEVIWTSSDHQ VLSGKTTTTNSKREEKLLNVTSTLRINTTANEIFYCIFRRL DPEENHTAELVIPELPLALPPNERTH |
| 13. | Rat PDL1 ECD | FTITAPKDLYVVEYGSNVTMECRFPVEQKLDLLALVVY WEKEDKEVIQFVEGEEDLKPQHSSFRGRAFLPKDQLLKG NAVLQITDVKLQDAGVYCCMISYGGADYKRITLKVNAP YRKINQRISMDPATSEHELMCQAEGYPEAEVIWTNSDHQ SLSGETTVTTSQTEEKLLNVTSVLRVNATANDVFHCTFW RVHSGENHTAELIIPELPVPRLPHNRTH |
| 14. | Mouse PDL1 ECD | FTITAPKDLYVVEYGSNVTMECRFPVERELDLLALVVYW EKEDEQVIQFVAGEEDLKPQHSNFRGRASLPKDQLLKGN AALQITDVKLQDAGVYCCIISYGGADYKRITLKVNAPYR KINQRISVDPATSEHELICQAEGYPEAEVIWTNSDHQPVS GKRSVTTSRTEGMLLNVTSSLRVNATANDVFYCTFWRS QPGQNHTAELIIPELPATHPPQNRTH |
| 15. | Heavy chain of SGN-PDL1V antibody moiety | QVQLVQSGAEVKKPGSSVKVSCKTSGDTFSTAAISWVRQ APGQGLEWMGGIIPIFGKAHYAQKFQGRVTITADESTSTA YMELSSLRSEDTAVYFCARKFHFVSGSPFGMDVWGQGT TVTVSSASTKGPSVFPLAPSSKSTSGGTAALGCLVKDYFP EPVTVSWNSGALTSGVHTFPAVLQSSGLYSLSSVVTVPSS SLGTQTYICNVNHKPSNTKVDKKVEPKSCDKTHTCPPCP APEAAGGPSVFLFPPKPKDTLMISRTPEVTCVVVDVSHED PEVKFNWYVDGVEVHNAKTKPREEQYNSTYRVVSVLTV LHQDWLNGKEYKCKVSNKALPAPIEKTISKAKGQPREPQ VYTLPPSRDELTKNQVSLTCLVKGFYPSDIAVEWESNGQ PENNYKTTPPVLDSDGSFFLYSKLTVDKSRWQQGNVFSC SVMHEALHNHYTQKSLSLSPGK |
| 16. | Light chain of SGN-PDL1V antibody moiety | EIVLTQSPATLSLSPGERATLSCRASQSVSSYLAWYQQKP GQAPRLLIYDASNRATGIPARFSGSGSGTDFTLTISSLEPE DFAVYYCQQRSNWPTFGQGTKVEIKRTVAAPSVFIFPPSD EQLKSGTASVVCLLNNFYPREAKVQWKVDNALQSGNSQ ESVTEQDSKDSTYSLSSTLTLSKADYEKHKVYACEVTHQ GLSSPVTKSFNRGEC |

### Examples

The present invention will be further described in the following examples, which should not be construed as a limitation to the present invention.

### Reagents and materials:

Organic solvents such as dimethyl sulfoxide were purchased from Spectrum Chemical Mfg Corp, and TCEP-HCl reagent from Thermo Fisher, etc.

IgG1 is an isotype control purchased from Beijing Sino Biological, Inc.

pH-sensitive Zenon^{™} pHrodo^{™} iFL IgG labeling reagent was purchased from Thermo Fisher.

CellTiter-Glo solution was purchased from Promega.

Cell strains NCI-H292 , BXPC-3 (for in vivo models), and MDA-MB-231 were purchased from the Cell Bank of the Chinese Academy of Sciences, NCI-H441 was purchased from ATCC, BXPC-3 (for in vitro cell killing) was purchased from Wuhan Pricella Biotechnology Co., Ltd., and various PDX transplants were established and maintained by Shanghai Lide Biotech Co., Ltd.

### Preparation scheme:

The structures of compounds described in the following examples were determined by nuclear magnetic resonance hydrogen spectroscopy (¹HNMR ) or liquid chromatography-mass spectrometry ( LC-MS ).

Bruker 400 MHz nuclear magnetic resonance instrument was used for the measurement of nuclear magnetic resonance hydrogen spectroscopy (¹HNMR); the solvent for measurement was deuterated methanol (CD3OD), deuterated chloroform (CDCl3), or hexadeuterated dimethyl sulfoxide (DMSO-d6); and the internal standard substance is tetramethylsilane (TMS).

The abbreviations in nuclear magnetic resonance (NMR) spectra used in the examples are shown below.

s: singlet; d: doublet; t: triplet; q: quartet; dd: double doublet; qd: quarter doublet; ddd: double doubledoublet; ddt: double doubletriplet; dddd: double doubledouble doublet; m: multiplet; br: broad; J: coupling constant; Hz: Hertz; DMSO-d6: deuterated dimethyl sulfoxide. Δ values were expressed in ppm.

Detection by liquid chromatography-mass spectrometry (LC-MS) was carried out using model Agilent 6120B Agilent (ESI) mass spectrometer.

### Example 1. Preparation of linker-toxin (LP62)

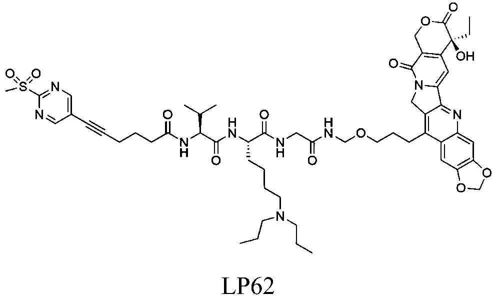

### Example 1.1: Synthesis of (S)-7-ethyl-7-hydroxy-14-(3-hydroxypropyl)-10,13-dihydro-11H[1,3]dioxolo[4,5-g]pyrano[3',4',6,7]indolizino[1,2-b]quinoline-8,11(7H)-dione (A1.3)

The synthesis route for compound A1.3 is as shown in scheme 1 below:

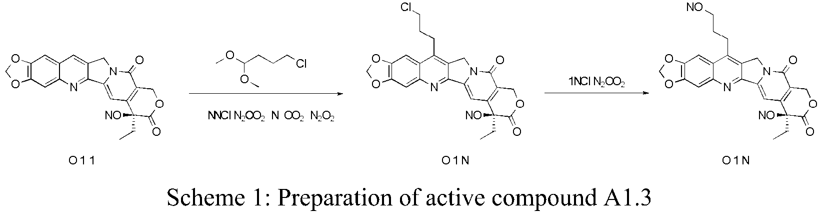

The specific steps were as follows:

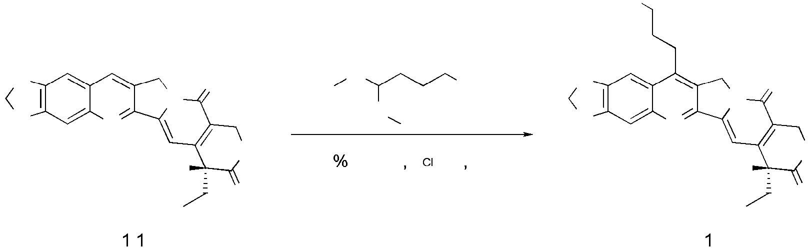

Step 1: Under ice bath conditions, to a solution of (*S*)-7-ethyl-7-hydroxy-10,13-dihydro-11*H*-[1,3]dioxolo[4,5-*g*]pyrano[3',4',6,7]indolizino[1,2-*b*]quinoline-8,11(7*H*)-dione (**A1.1**, 500 mg) in 75% sulfuric acid (5 mL) was added ferrous sulfate heptahydrate (570 mg of ferrous sulfate heptahydrate dissolved in 1 mL of water) and 4,4-dimethoxychlorobutane (3.89 g). The reaction liquid was stirred for three minutes, and hydrogen peroxide (29%, 2.5 mL) was then dropwise added under ice bath conditions. After the dropwise addition was complete, the reaction liquid was reacted under stirring at 0°C for 5 minutes, then warmed to room temperature, and reacted under stirring for 3 hours. The reaction liquid was diluted with water (50 mL) and extracted with ethyl acetate (80 mL × 2). The organic phase was washed with a saturated aqueous sodium chloride solution and then dried over anhydrous sodium sulfate. The organic phase was evaporated under reduced pressure and then concentrated to give a crude product, which was purified using preparative liquid chromatography equipped with a C18 column (acetonitrile/0.05% aqueous formic acid solution; 5%-60%) to finally give the target compound (**A1.2**, as a yellow solid, 400 mg, yield: 67%).

LC-MS (ESI) [M+H]⁺: 468.9:
¹HNMR (400 Hz, DMSO-*d6*) δ7.65 (s,1H), 7.51 (s,1H),7.24 (s, 1H), 6.50 (s,1H), 6.30 (s, 2H), 5.42 (s, 2H), 5.26 (s, 2H), 3.81(d, *J =* 5.9 Hz, 2H), 3.22 (s, 2H), 1.98 (d, *J =* 6.7 Hz, 4H), 0.88(t, *J =* 7.2 Hz, 3H).

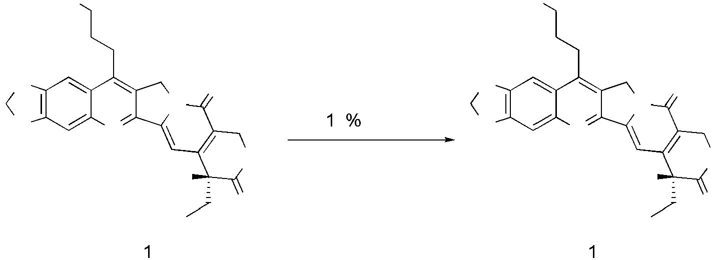

Step 2: (S)-7-ethyl-7-hydroxy-14-(3-chloropropyl)-10,13-dihydro-11H-[1,3]dioxolo[4,5-g]pyrano[3',4',6,7]indolizino[1,2-b]quinoline-8,11(7H)-dione (A1.2, 100 mg, 0.213 mmol) was dissolved in a 10% sulfuric acid solution (5 mL), and the reaction liquid was reacted at 110°C for 48 hours. To the reaction liquid was added a saturated sodium bicarbonate solution (30 mL), and the reaction liquid was extracted with dichloromethane (10 mL × 5), dried over anhydrous sodium sulfate, suction-filtered, and concentrated under reduced pressure to give a crude product. The crude product was subjected to preparative purification by high performance liquid chromatography (acetonitrile/water, with 0.05% formic acid) to give (S)-7-ethyl-7-hydroxy-14-(3-hydroxypropyl)-10,13-dihydro-11H-[1,3]dioxolo[4,5-g]pyrano[3',4',6,7]indolizino[1,2-b]quinoline-8,11(7H)-dione (**A1.3**, 1.78 mg).

LC-MS (ESI) [M+H]⁺: 451.0:
1HNMR (400 Hz, DMSO-*d6*) δ7.63 (s,1H), 7.50 (s,1H),7.24 (s, 1H), 6.48 (s,1H), 6.28 (s, 2H), 5.47-5.37 (m, 2H), 5.32-5.19 (m, 2H), 3.51-3.46 (m, 2H), 3.17-3.13 (m, 2H), 1.92-1.76 (m, 4H), 0.90-0.84 (m, 3H).

### Example 1.2: Synthesis of compound A2.3

The synthesis route for compound A2.3 is as shown in scheme 2 below:

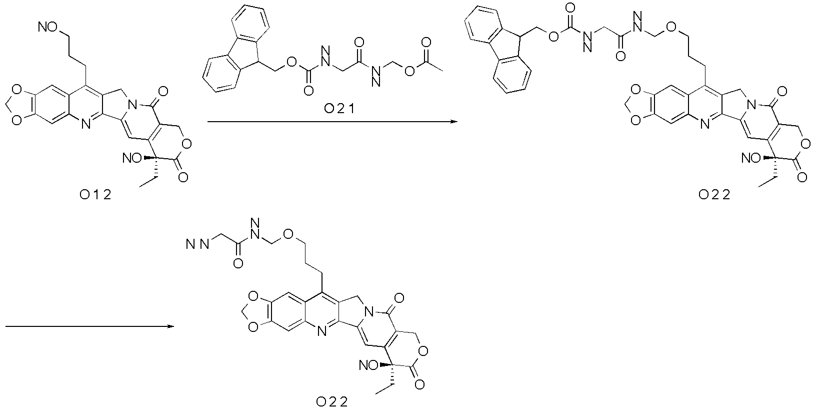

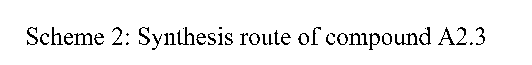

The specific steps for the synthesis of compound A2.3 were as follows:

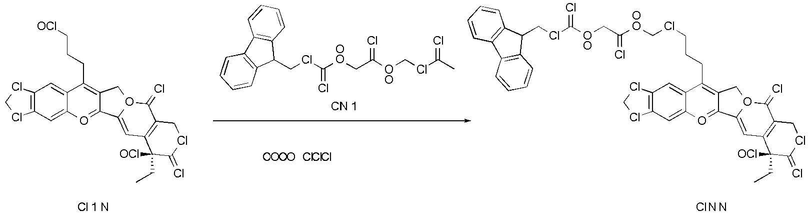

Step 1: Compound A2.1 (368 mg), compound A1.3 (440 mg), and pyridinium p-toluenesulfonate (PPTS, 25 mg) were refluxed in dichloromethane (20 mL) at 40°C for 20 hours and then washed separately with an aqueous sodium bicarbonate solution and an aqueous hydrochloric acid solution, and the organic solvent was removed under reduced pressure to give a crude product. The crude product was separated and purified by column chromatography (dichloromethane:methanol = 10 : 1) to give the target compound A2.2 (240 mg).

LC-MS (ESI) [M+H]⁺: 747:

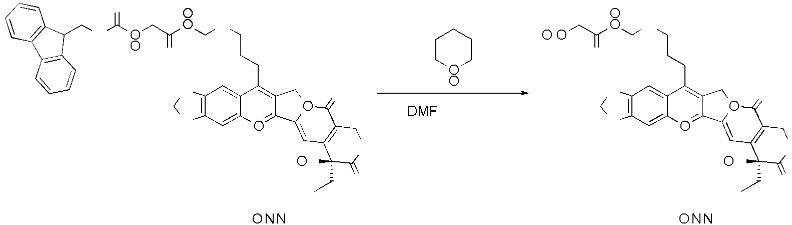

Step 2: A2.2 (240 mg) was dissolved in DMF (5 mL), and piperidine (1 mL) was added. The mixture was stirred at room temperature for 20 minutes. Low-boiling-point components were removed under pressure, and the residue was used directly in the next step of synthesis. A small amount of the crude product was purified by reverse-phase chromatography (acetonitrile/0.05% FA in water: 5%-50%) to give the target compound A2.3.

LC-MS (ESI) [M+H]⁺: 525:
¹H NMR (400 Hz, DMSO-d6) δ9.13 (t,1H), 8.04 (br, 2H), 7.58 (s, 1H), 7.51 (s,1H), 7.25 (s, 1H), 6.29 (s, 2H), 5.43 (s, 2H), 5.21 (s, 2H), 4.65 (d, 2H), 3.63 (m, 2H), 3.53 (m, 2H), 3.11 (m, 2H), 1.87 (m, 4H), 0.88 (t, 3H).

### Example 1.3: Synthesis of compound A3.5

The synthesis route for compound A3.5 is as shown in scheme 3 below:

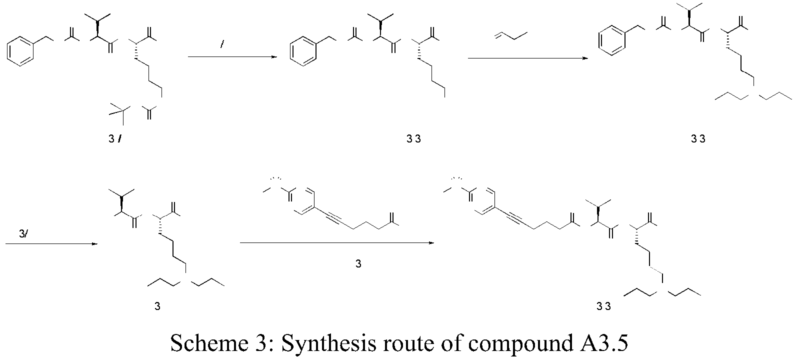

The specific synthesis steps for compound A3.5 were as follows:

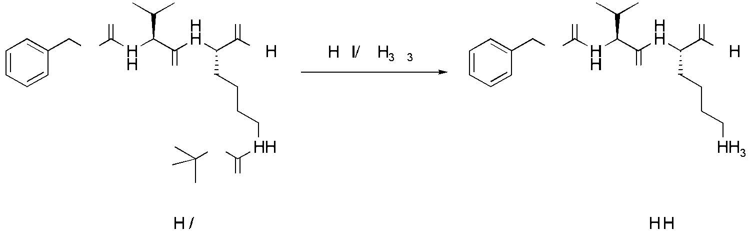

Step 1: To compound A3.1 (10.0 g) was added a solution of hydrogen chloride-dioxane (dioxane, C₄H₈O₂) (100 mL), and the mixture was reacted at room temperature for 2 hours. The reaction liquid was concentrated under reduced pressure to give the target compound A3.2 (8.0 g) as a white solid.

LC-MS (ESI) [M+H]⁺: 380.1
¹H NMR (400 MHz, DMSO-*d6*) δ 8.18 (d, *J =* 7.4 Hz, 1H), 7.40-7.30 (m, 5H), 7.26 (d, *J =* 8.8 Hz, 1H), 5.08-4.99 (m, 2H), 4.23-4.11 (m, 1H), 3.94-3.88 (m, 1H), 2.78-2.73 (m, 2H), 2.03-1.94 (m, 1H), 1.77-1.51 (m, 4H), 1.44-1.31 (m, 2H), 0.87 (dd, *J =* 17.3, 6.6 Hz, 6H).

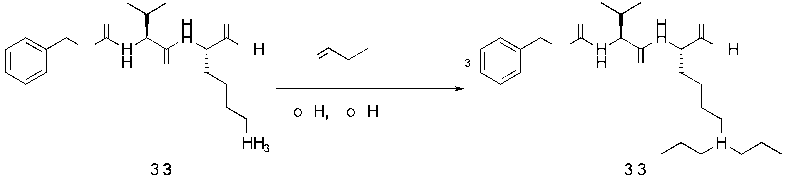

Step 2: Compound A3.2 (5.0 g, 12 mmol) was dissolved in dichloromethane (100 mL), and n-propanal (4.2 g, 72.3 mmol) was added to the reaction liquid. The reaction was stirred at room temperature for 10 minutes, and sodium triacetoxyborohydride (12.8 g, 60.25 mmol) was then added to the reaction liquid. The reaction was stirred at room temperature for 1 hour. LC-MS showed that the reaction was complete. A saturated aqueous ammonium chloride solution was added to the reaction liquid and stirred for 1 hour. After rotary drying and filtration, the filtrate was purified by reverse separation using a C18 column to give the target compound A3.3 (4.57 g, yield 82.0%). The compound A3.3 was a white solid.

LC-MS (ESI) [M+H]⁺: 464.0:
¹H NMR (400 MHz, DMSO-*d6*) δ 7.81 (d, *J* = 7.3 Hz, IH), 7.38-7.28 (m, 5H), 5.04 (d, *J* = 1.7 Hz,2H),4.12- 4.02 (m, IH), 3.94-3.82 (m, 1H), 2.65-2.52 (m, 6H), 2.06-1.94 (m, 1H), 1.76-1.64 (m, 1H), 1.64-1.53 (m, 1H), 1.52-1.40 (m, 6H), 1.34-1.18 (m, 2H), 0.92-0.80 (m, 12H).

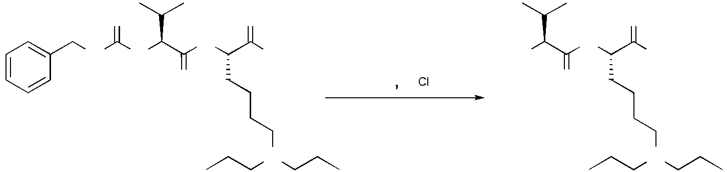

Step 3: Compound 3.3 (2.0 g, 4.32 mmol) was dissolved in methanol (80 mL) at room temperature, and Pd/C (0.16 g) was then added to the reaction liquid. The reaction was stirred at room temperature under hydrogen for 12 hours. After LC-MS showed that the reaction was complete, the reaction liquid was filtered and concentrated under reduced pressure to give the target compound A3.4 (1.2 g, yield 85.5%) as a white solid.

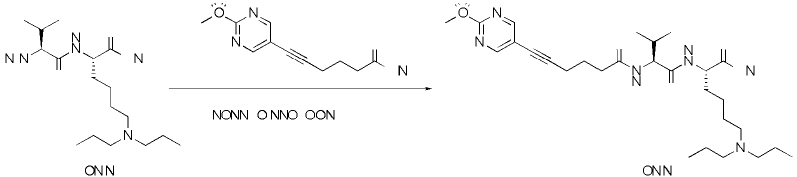

Step 4: 6-(2-(Methylsulfonyl)pyrimidin-5-yl)hex-5-ynoic acid (100 mg, 0.373 mmol) was dissolved in DMF (1 mL), and HATU (142 mg, 0.373 mmol) and *N,N-*diisopropylethylamine (120 mg, 0.93 mmol) were then added. The system was stirred for 30 minutes, and compound A3.4 (122 mg, 0.371 mmol) was then added. The reaction liquid was stirred at room temperature for 1 hour. After the reaction was complete as detected by LC-MS, the reaction liquid was directly purified by reverse phase chromatography on a C18 column (acetonitrile and a 0.05% formic acid aqueous solution system) to obtain the target compound N⁶,N⁶-dipropyl-N²-((6-(2-(methylsulfonyl)pyrimidin-5-yl)hex-5-ynoyl)-L-valine)-L-lysine (compound A3.5, 50 mg, yield 28%) as a pale yellow solid.

LC-MS (ESI) [M+H]⁺: 580.0:
1HNMR (400 MHz, DMSO-*d6*) δ 8.24 (s, 2H), 7.98-7.93 (m, 2H), 4.24-4.16 (m, 1H), 4.10 (d, *J =* 5.2Hz, 1H), 3.41 **(s**, 3H), 2.79-2.64 (m, 6H), 2.55 (t, *J =* 7.1Hz, 2H), 2.45-2.26 (m, 2H), 2.06-1.91 (m, 1H),1.89-1.78 (m, 2H), 1.76-1.66 (m, 1H), 1.64-1.57 (m, 1H), 1.57-1.42 (m, 6H). 1.37-1.24 (m, 2H), 0.93-0.78 (m, 12H).

### Example 1.4: Synthesis of linker-toxin (LP62)

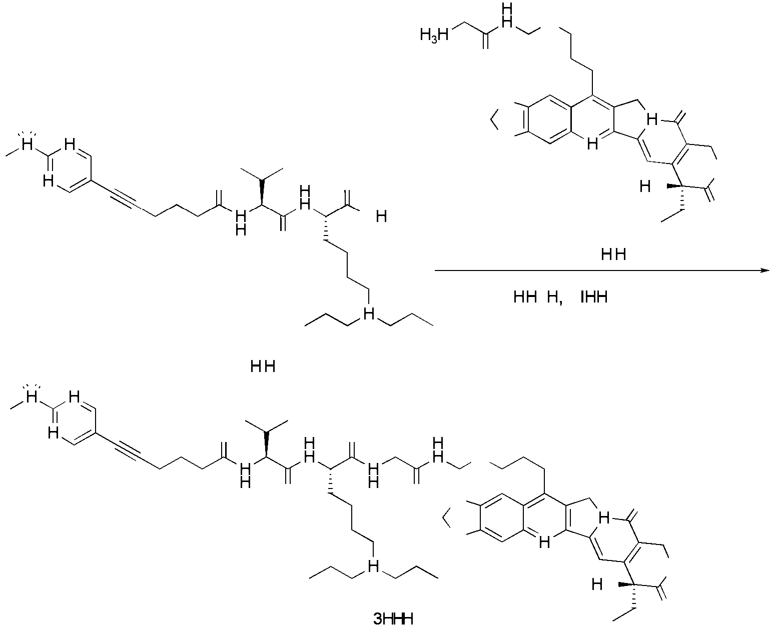

Step 1: Compound A3.5 (43 mg, 0.074 mmol) and compound A2.3 (40 mg, 0.074 mmol) were dissolved in 1 mL of DMF, and HBTU (28 mg, 0.075 mmol) and *N*,*N*-diisopropylethylamine (24 mg, 0.187 mmol) were then added sequentially. The reaction liquid was stirred at room temperature for 1 hour. After the reaction was complete as detected by LC-MS, the reaction liquid was directly purified by preparative chromatography (a 0.01% trifluoroacetic acid aqueous solution, acetonitrile) to give the target compound LP62 as a trifluoroacetate salt (8.5 mg, yield 10%) as a yellow solid.

LC-MS (ESI) [M+H]⁺: 1098.6:
¹H NMR (400 MHz, DMSO-*d₆*): δ 9.10 (s, 2H), 9.03 (s, 1H), 8.64 (t, *J* = 6.4 Hz, 1H), 8.19 (m, *J* = 5.9 Hz, 1H), 8.08 (d, *J* = 7.4 Hz,1H), 7.92 (d, *J* = 8.5 Hz,1H), 7.59 (s, 1H), 7.51 (s, 1H), 7.24 (s, 1H), 6.49 (s, 1H), 6.29 (s, 2H), 5.42 (s, 2H), 5.24 (s, 2H), 4.66-4.52 (m, 2H), 4.31-4.21 (m, 1H), 4.19-4.10 (m, 1H), 3.74 (d, *J =* 5.5 Hz, 2H), 3.49-3.48 (m,2H),3.40 (s, 3H), 3.15-3.06 (m,2H), 3.02-2.95 (m,6H), 2.59-2.52 (m, 3H), 2.41-2.29 (m, 2H), 2.50-1.90 (m, 2H), 1.91-1.77 (m, 6H), 1.63-1.57 (m, 6H), 1.31-1.29 (m,2H), 0.92-0.80 (m,15H).

### Example 2. Preparation of antibody

Unless otherwise indicated, the heavy chain and light chain amino acid sequences of the anti-PDL1 antibody used in the present invention are described in the Sequence Listing.

In general, the antibody was prepared as follows: A stable CHO cell line expressing and secreting an anti-PDL1 antibody was constructed. The stable CHO cell line was cultured in a suitable culture medium. A culture supernatant was harvested and subjected to sequentially depth filtration, protein A column affinity chromatography, virus inactivation, filtration, anion chromatography, cation chromatography, nanofiltration/ultrafiltration, and other processes. Finally, a target antibody was obtained.

### Example 3. Preparation and detection of antibody-drug conjugate

### Example 3.1. Preparation of MAB20-LP62

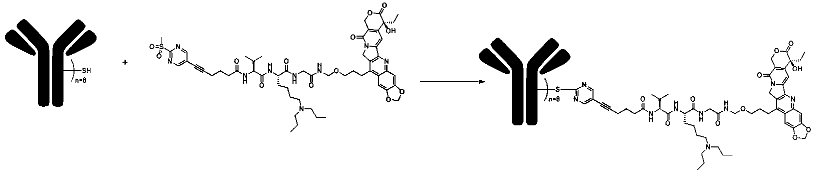

wherein n = 8, indicating the molar ratio of the linker-loaded drug to the antibody.

2-5mL of the anti-PDL1 antibody MAB20 (Anti-PDL1_mAb, 10-30 mg/mL) was placed in a dedicated antibody conjugation reactor. EDTA at a final concentration of 10-20 mM was added, and the pH was then adjusted to 5.5-7.5 with a 0.5-1 M disodium hydrogen phosphate solution. 10-20 mM TCEP (tris(2-carboxyethyl)phosphine, 4-10 eq., 0.1-0.8 mL) was slowly added, and the mixture was mixed uniformly and reacted at room temperature for 50-80 minutes. After the reduction reaction of the anti-PDL1 antibody was complete, a solution of linker-toxin LP62 (8-15 eq., 2-10 µmol) previously dissolved in dimethylsulfoxide (DMSO) or DMAC solvent was added to the above solution system, and the system was stirred and mixed uniformly and reacted at room temperature for 100-150 minutes. After purification by cation chromatography, the system buffer was then replaced with 20-50 mM PB, pH 5.5-7.5, by using Amicon Ultra-15 to give MAB20-YL62, a conjugate product of the anti-PDL1 antibody MAB20 and the linker-toxin LP62. The DAR value and monomer content thereof were verified by LC-MS, HIC, and HPLC-SEC analysis and met the standards.

### Example 3.2. Preparation of reference ADC MAB20-VC-MMAE

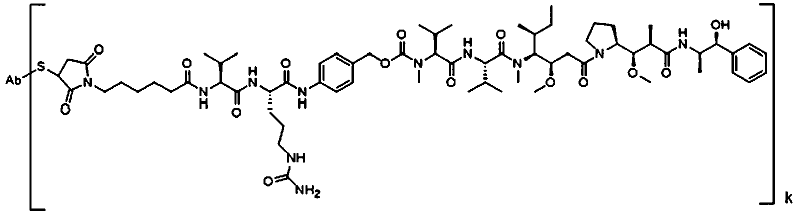

### Structure of MAB20-VC-MMAE (in which Ab was MAB20, and k was the DAR value)

MAB20-VC-MMAE was prepared according to a general method in the prior art. Specifically, 2-5 mL of the PDL1 antibody MAB20 (Anti-PDL1_mAb, 10-30 mg/mL) was placed in a dedicated antibody conjugation reactor. EDTA at a final concentration of 10-20 mM was added, and the pH was then adjusted to 5.5-7.5 with a 0.5-1 M disodium hydrogen phosphate solution. 10-20 mM TCEP (tris(2-carboxyethyl)phosphine, 2-4 eq., 0.1-0.8 mL) was slowly added, and the mixture was mixed uniformly and reacted at room temperature for 50-80 minutes. After the reduction reaction of MAB20 was complete, a solution of linker-toxin MC-VC-PAB-MMAE (the structure of which was as shown below) (4-15 eq., 1-10 µmol) previously dissolved in a dimethylsulfoxide (DMSO) or DMAC solvent was added to the above solution system, and the system was stirred and mixed uniformly and placed at room temperature for 100-150 minutes. After purification by cation chromatography, the system buffer was replaced with 20-50 mM PB, pH 5.5-7.5, by using Amicon Ultra-15 to give MAB20-VC-MMAE, a conjugate product of the anti-PDL1 antibody MAB20 and MC-VC-PAB-MMAE. The DAR value and monomer content thereof were verified by LC-MS, HIC, and HPLC-SEC analysis and met the standards.

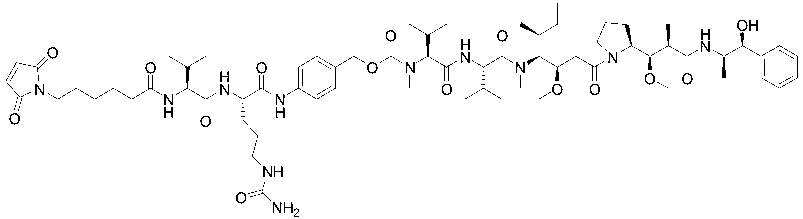

### MC-VC-PABC-MMAE

| Sample name | Aggregate (%) | Main peak (%) | Fragment (%) |
|---|---|---|---|
| MAB20-VC-MMAE | 0.8 | 98.6 | 0.6 |

| Sample name | Chain | DAR for LC/HC | DAR for IgG |
|---|---|---|---|
| MAB20-VC-MMAE | LC (light chain) | 0.6 | 4.00 |
| | HC (heavy chain) | 1.40 | |

### DAR value for anti-PDL1 antibody-drug conjugate sample

### Example 3.3. Preparation of reference ADC SGN-PDL1V

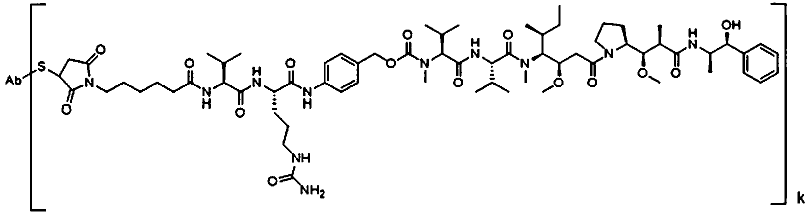

### Structure of SGN-PDL1V (in which Ab was the antibody moiety of SGN-PDL1V, and k was the DAR value)

SGN-PDL1V can be prepared by reference to the method described in WO 2021067776 A1. Alternatively, it can be prepared as follows: 2-5 mL of the antibody moiety of SGN-PDL1V (10-30 mg/mL) was placed in a dedicated antibody conjugation reactor. EDTA at a final concentration of 10-20 mM was added, and the pH was then adjusted to 5.5-7.5 with a 0.5-1 M disodium hydrogen phosphate solution. 10-20 mM TCEP (tris(2-carboxyethyl)phosphine, 2-4 eq., 0.1-0.8 mL) was slowly added, and the mixture was mixed uniformly and reacted at room temperature for 50-80 minutes. After the reduction reaction of the antibody was complete, a solution of linker-toxin MC-VC-PAB-MMAE (the structure of which was as shown below) (4-15 eq., 1-10 µmol) previously dissolved in a dimethylsulfoxide (DMSO) or DMAC solvent was added to the above solution system, and the system was stirred and mixed uniformly and placed at room temperature for 100-150 minutes. After purification by cation chromatography, the system buffer was replaced with 20-50 mM PB, pH 5.5-7.5, by using Amicon Ultra-15 to give SGN-PDL1V, a conjugate product of the antibody moiety of SGN-PDL1V and MC-VC-PAB-MMAE. The DAR value (DAR4) and monomer content thereof were verified by LC-MS, HIC, and HPLC-SEC analysis and met the standards.

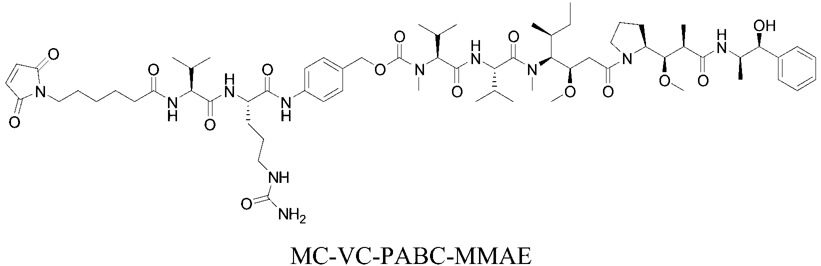

### Example 3.4. Preparation of reference ADC MAB20-GGFG-DXD

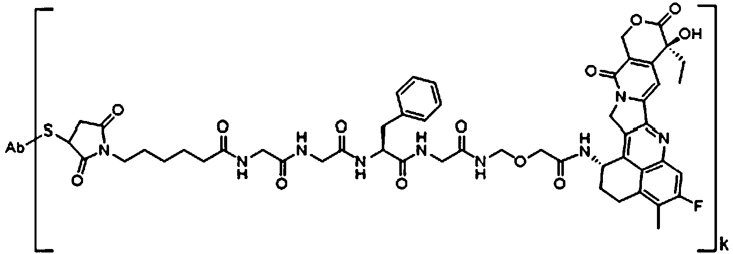

### Structure of MAB20-GGFG-DXD (in which Ab was MAB20, and k was the DAR value)

MAB20-GGFG-DXD was prepared according to a general method in the prior art. Specifically, 2-5 mL of the anti-PDL1 antibody MAB20 (Anti-PDL1_mAb, 10-25 mg/mL) was taken and placed in a reaction tube. EDTA at a final concentration of 10-20 mM was added, and the pH was then adjusted to 6.0-7.5 with a 0.5-1 M disodium hydrogen phosphate solution. 10-20 mM TCEP (tris(2-carboxyethyl)phosphine, 4-10 eq., 0.1-0.4 mL) was slowly added, and the mixture was mixed uniformly and reacted at room temperature for 50-80 minutes. After the reduction reaction of MAB20 was complete, a solution of linker-toxin MC-GGFG-DXD (the structure of which was as shown below) (8-12 eq., 2-10 µmol) previously dissolved in a dimethylsulfoxide (DMSO) or DMAC solvent was added to the above solution system, and the system was stirred and mixed uniformly and placed at room temperature for 100-150 minutes. The system buffer was replaced with 10-50 mM PB or histidine-histidine hydrochloride, pH 5.5-7.5, by using Amicon Ultra-15 to give MAB20-GGFG-DXD, a conjugate product of MAB20 and the linker-toxin MC-GGFG-DXD. The DAR value and monomer content thereof were verified by LC-MS, HIC, and HPLC-SEC analysis and met the standards.

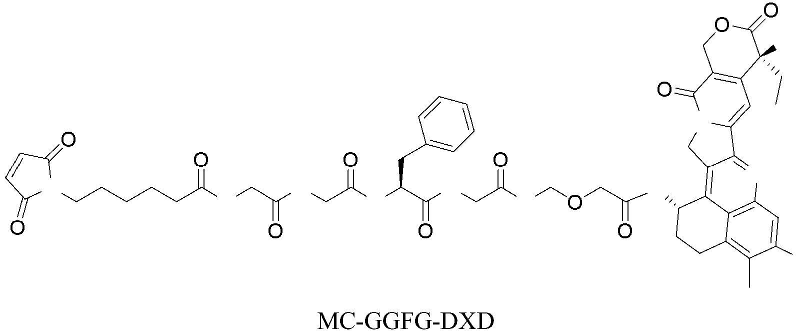

### Example 3.5: Analysis of antibody-drug conjugate by LC-MS for loaded drug : antibody ratio (DAR)

An antibody-drug conjugate sample was reduced with DTT at 37°C for 10 minutes, and the DAR value of the conjugate was then analyzed using LC-MS. Specifically, the reduced sample was injected into an LC-MS system (Agilent 1290 Infinity II LC system and Agilent 6545XT AdvanceBio Q-TOF) equipped with an Acquity UPLC BEH C4 reverse phase column (Waters Corporation, 2.1 mm × 50 mm, 1.7 µm, 300 Å). The drug : antibody ratio (DAR) was calculated using the provided deconvoluted LC-MS results.

### Example 4. Assay for affinity of anti-PDL1 antibody-drug conjugate for PDL1-positive cells

Normally grown and passaged NCI-H292 and NCI-H441 tumor cells were respectively collected, washed with PBS, resuspended, and inoculated into a 96-well plate at 1 × 10⁶ cells/well/50 µl. The test sample was then diluted with a gradient using, for example, an FACS buffer (PBS + 2% FBS), and after dilution, the test sample to be tested and the control sample were added sequentially to the 96-well plate inoculated with the cells, wherein the control sample IgG1 was a human IgG1, κ isotype control (purchased from Beijing Sino Biological, Inc.). The sample plate was incubated at 4°C for 0.5 h and centrifuged, followed by supernatant removal and washing with 300 µl/well of FACS buffer. Subsequently, a fluorescently labeled goat anti-human IgG antibody detection solution was added to the sample plate (100 µl/well) in a sequential manner for incubation with the cells. The sample plate was incubated at 4°C for 0.5 h and washed with 300 µl/well of FACS buffer, and finally, the cells were resuspended. The fluorescence numerical value for each sample was detected by a flow cytometer. The data were analyzed on Graphpad prism 8.1 and fitted to a four-parameter equation.

As shown in FIG. 1A, MAB20-LP62 (DAR8) exhibited better affinity for PDL1-positive cells NCI-H292, and the affinity thereof was comparable to the affinity of the unconjugated antibody MAB20. Similarly, as shown in FIG. 1B, MAB20-LP62 (DAR8) exhibited better affinity for PDL1-positive cells NCI-H441.

### Example 5. Assay for internalization of anti-PDL1 antibody-drug conjugate into PDL1-positive cells

A volume of normally grown and passaged NCI-H292 cells was taken and centrifuged, followed by supernatant removal and resuspension in a cell culture medium (RPMI-1640 culture medium + 10% FBS). 50 µl of the cell suspension was inoculated into a 96-well plate for use (1 × 10⁵ cells/well). A pH-sensitive Zenon^{™} pHrodo^{™} iFL IgG labeling reagent (purchased from ThermoFisher) was formulated with the cell culture medium to form 4x working solution 1 (480 nM). This pH-sensitive dye conjugate did not fluoresce extracellularly, but fluoresced brightly in an acidic environment, including lysosomes. The test material was formulated with the cell culture medium to form 4x working solution 2 (160 nM). 25 µl of working solution 1 and 25 µl of working solution 2 were taken separately and added to a 96-well plate, followed by mixing and incubation. The mixed liquid was pipetted into 50 µl of the cell suspension, mixed uniformly, and placed in an incubator for culturing. Test materials were taken at 0, 2, 16, 24 h time points and centrifuged, and the supernatants were removed. The resulting material was then washed with 300 µl/well of FACS buffer and resuspended, and the fluorescence value of each sample was measured using a flow cytometer.

As shown in FIG. 2, as compared with the data of the IgG1 isotype control, the proportion of MAB20-LP62 (DAR8) and MAB20 internalization increased as the incubation time increased and reached the peak at 4 h, indicating that the anti-MAB20 antibody-drug conjugate MAB20-LP62 (DAR8) can effectively bind the PDL1 antigen on the cell surface and be internalized into the intracellular lysosomal pathway.

### Example 6. Assay for cell killing activity of anti-PDL1 antibody-drug conjugate

Cells in exponential growth phase are collected and viable cells are counted using a Vi-Cell XR cytometer. The cell suspension was adjusted to an appropriate concentration with a culture medium. 80 µl/well of the cell suspension was added to a cell culture plate. The test sample was diluted with a gradient (5-fold dilutions). 20 µl of each of the diluted test articles was taken and added to the cell suspension in the cell culture plate, and the cell culture plate was placed in an incubator and cultured for 72 hours. After 72 hours, 50 µl/well of a CellTiter-Glo solution (purchased from Promega) that had been thawed in advance and equilibrated to room temperature was added according to CellTiter-Glo operation instructions. The wells were mixed uniformly with a microwell plate shaker for 2 minutes and maintained at room temperature for 10 minutes. The fluorescence signal was measured using Envision 2104 plate reader. The raw data were analyzed using Graphpad prism 8.1 and fitted to a four-parameter equation.

As shown in FIG. 3, MAB20-LP62 (DAR8) showed stronger cell killing effects on MAB20-insensitive PDL1-positive tumor cells NCI-H292, BXPC-3, and NCI-H441.

### Example 7. Cross-binding activity of anti-PDL1 antibody-drug conjugate to PDL1s from different species

Extracellular domains (ECDs) of PDL1s derived from human, cynomolgus monkey, rat, and mouse were separately diluted with 1×PBS to a working concentration of 2 µg/mL, as ELISA coating solutions. The test plate was washed 3 times with PBST (PBS with 0.05% Tween 20). The plate was incubated with a blocking solution SuperBlock T20 (PBS) Blocking Buffer (60 µL/well) at room temperature, the test sample was then diluted with a gradient, and the diluted samples were then loaded separately into a test plate and incubated at room temperature. The test plate was washed, and HRP enzyme-labeled goat anti-human IgG was added as a secondary antibody, followed by incubation. The test plate was washed, and a stop solution (30 µL/well) was added, followed by centrifugation. The light absorbance at a wavelength of 450 nm was read. The raw data were analyzed using Graphpad prism 8.1 and fitted to a four-parameter equation.

As shown in FIG. 4, the binding affinity of MAB20-LP62 for cynomolgus and mouse PDL1 ECDs was close to that for human PDL1 ECD, with EC50 of 0.039 nM, 0.094 nM, and 0.054 nM, respectively. MAB20-LP62 (DAR8) showed weak specific binding to rat PDL1 ECD.

### Example 8. Assay for bystander effect of anti-PDL1 antibody-drug conjugate

PDL1-negative tumor MDA-MB-468 cells in the exponential growth phase were collected, and viable cells were counted using Vi-Cell XR cytometer. The cell suspension was adjusted to an appropriate concentration with a culture medium. 80 µl/well of the cell suspension was added to a cell culture plate. The test drug was diluted with a gradient (5-fold dilutions). 20 µl of each of the diluted test articles was taken and added to the cell suspension in the cell culture plate, and the cell culture plate was placed in an incubator and cultured for 96 hours. 50 µl/well of a CellTiter-Glo solution (purchased from Promega) that had been thawed in advance and equilibrated to room temperature was added according to CellTiter-Glo operation instructions. The wells were mixed uniformly with a microwell plate shaker for 2 minutes and maintained at room temperature for 10 minutes. The fluorescence signal was measured using Envision 2104 plate reader.

PDL1-overexpressing HEK293 cells in the exponential growth phase were collected, and viable cells were counted using Vi-Cell XR cytometer. The cell suspension was adjusted to an appropriate concentration with a culture medium. 80 µl/well of the cell suspension was added to a cell culture plate. The test drug was diluted with a gradient (5-fold dilutions). 20 µl of each of the diluted test articles was taken and added to the cell suspension in the cell culture plate, and the cell culture plate was placed in an incubator and cultured for 96 hours. After 96 hours, a conditioned medium supernatant from PDL1-overexpressing HEK293 cells was collected and transferred in an equal volume for culturing MDA-MB-468 cells. After continued culturing for 96 hours, 50 µl/well of a CellTiter-Glo solution (purchased from Promega) that had been thawed in advance and equilibrated to room temperature was added according to CellTiter-Glo operation instructions. The wells were mixed uniformly with a microwell plate shaker for 2 minutes and maintained at room temperature for 10 minutes. The fluorescence signal was measured using Envision 2104 plate reader.

As shown in FIGS. 5A and 5B, MAB20-LP62 (DAR8) had significant bystander effect.

### Example 9. Assay for immunotoxicity of anti-PDL1 antibody-drug conjugate

Since some human immune cells such as antigen-presenting cells (macrophages, dendritic cells, etc.) express PDL1, the immunotoxicity of PDL1-targeted antibody-conjugated drugs is an inevitable problem. Human dendritic cells and macrophages, after induced differentiation, were digested and viable cells were counted using Vi-Cell XR cytometer. The cell suspension was adjusted to an appropriate concentration with a culture medium. 80 µl/well of the cell suspension was added to a cell culture plate, which was supplemented with 500 IU/mL of IFN-γ. The test drug was diluted with a gradient (5-fold dilutions). 20 µl of each of the diluted test articles was taken and added to the cell suspension in the cell culture plate, and the cell culture plate was placed in an incubator and cultured for 96 hours. 50 µl/well of a CellTiter-Glo solution (purchased from Promega) that had been thawed in advance and equilibrated to room temperature was added according to CellTiter-Glo operation instructions. The wells were mixed uniformly with a microwell plate shaker for 2 minutes and maintained at room temperature for 10 minutes. The fluorescence signal was measured using Envision 2104 plate reader. SGN-PDL1V was used as one of the control molecules and was disclosed in U.S. Patent No. US 20210101982 A1.

As shown in FIGS. 6 and 7, the control molecule SGN-PDL1V exhibited similar immunotoxicity in macrophages and dendritic cells in vitro as compared with the isotype control ADC (IgG1-MMAE); however, when the concentration was above 20 nM, SGN-PDL1V resulted in non-specific killing of both immune cells. In contrast, MAB20-LP62 (DAR8) did not show significant cytotoxicity to macrophages and dendritic cells even at high concentrations (500 nM). As a result, MAB20-LP62 had negligible immunotoxicity and higher safety as compared with SGN-PDL1V.

### Example 10. Anti-tumor activity of anti-PDL1 antibody-drug conjugate in NCG mouse model inoculated subcutaneously with human breast cancer MDA-MB-231 cells

MDA-MB-231 cells were cultured in a growth culture medium (RPMI 1640 + 10% FBS + IL15). Cells in the logarithmic growth phase were collected, washed twice with DPBS, and used for inoculation. The cells were resuspended with PBS and Matrixgel matrix at a ratio of 1:1, MDA-MB-231 cells (5 x 10⁶/100 µl/mouse) were inoculated subcutaneously on the right back of mice at the age of 6-7 weeks. Once the tumor had grown to an average volume of 204.54 mm³, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume (mm³) = 1/2 × (a × b²) (wherein a represented the long diameter, and b represented the short diameter).

As shown in FIG. 8, on day 35 post-dose, compared with the control group (MAB20, 8 mg/kg, tumor growth inhibition rate (TGI) 21.26%), MAB20-LP62 (DAR4) (3 mg/kg and 8 mg/kg) and MAB20-LP62 (DAR8) (3 mg/kg and 8 mg/kg) both showed significant tumor growth inhibition, with TGI of 46.89% (p < 0.001), 74.50% (p < 0.001), 80.33% (p < 0.001), and 92.91% (p < 0.001), respectively. MAB20-LP62 (DAR8) exhibited significant anti-tumor effects at low doses. Compared with the antibody-drug conjugates MAB20-VC-MMAE (DAR4) (3 mg/kg, TGI 38.79%) and MAB20-GGFG-DXD (DAR8) (3 mg/kg, TGI 44.60%; 8 mg/kg, TGI 72.98%) constructed based on a conjugation technique in the prior art, MAB20-LP62 (DAR8) showed significantly excellent anti-tumor activity. None of the animals experienced significant body weight loss throughout the treatment period.

### Example 11. Anti-tumor activity of anti-PDL1 antibody-drug conjugate in NCG mouse model inoculated subcutaneously with low-PDL1-expressing human pancreatic cancer BxPC3 cells

BXPC3 cells were cultured in a growth culture medium (RPMI 1640 + 10% FBS). Cells in the logarithmic growth phase were collected, washed twice with DPBS, and used for inoculation. The cells were resuspended with PBS and Matrixgel matrix at a ratio of 1:1. BXPC3 cells (5 x 10⁶/100 µl/mouse) were inoculated subcutaneously on the right back of mice at the age of 7-8 weeks. Once the tumor had grown to an average volume of 210.90 mm³, the mice were randomly grouped depending on the tumor size and dosed, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume (mm³) = 1/2 × (a × b2) (wherein a represented the long diameter, and b represented the short diameter).

As shown in FIG. 9, on day 35 post-dose, compared to the control group (MAB20) (TGI -28.31%), MAB20-LP62 (DAR4) (8 mg/kg) and MAB20-LP62 (DAR8) (8 mg/kg) both exhibited certain tumor inhibitory effects, with TGIs of 14% and 40.9%, respectively. Compared to the other test drugs MAB20-VC-MMAE (DAR4) (3 mg/kg, TGI -6.79%) and MAB20-GGFG-DXD (DAR8) (3 mg/kg, TGI -18.00%; 8 mg/kg, TGI -0.12%), only MAB20-LP62 achieved tumor growth inhibitory effect in models with low PDL1 expression. None of the animals experienced significant body weight loss throughout the treatment period.

### Example 12. Anti-tumor effect of anti-PDL1 antibody-drug conjugate in human non-small cell lung cancer LU6437 PDX xenograft BALB/c nude mice

A revived tumor tissue was harvested from HuPrime^{®} lung cancer xenograft model LU6437 tumor-bearing mice and cut into tumor blocks with a diameter of 2-3 mm, which were inoculated subcutaneously at the right anterior scapula of BALB/c nude mice. Once the tumor had grown to a volume of about 200 mm³, the mice were randomly grouped depending on the tumor size, ensuring that each group had a similar tumor volume. The average volume was 253.70 mm³ in the actual groups. The day of grouping was defined as day 0. After the start of dosing, the mice were dosed once a week, for three doses, and the body weight and tumor size of the mice were measured twice a week. Clinical symptoms observed during the experiment were recorded. The tumor volume was calculated according to the following formula: tumor volume (mm3) = 1/2 × (a × b2) (wherein a represented the long diameter, and b represented the short diameter).

It should be noted that the preliminary pre-experiment had showed severely lethal toxicity of SGN-PDL1V at a dose of 8 mg/kg to mice. For ethical considerations, the experimental group was not set in the experimental design.

As shown in FIG. 10, in the anti-PD1 monoclonal antibody-resistant human non-small cell lung cancer PDX model LU6437, MAB20-LP62 at 1 mg/kg (day 21, TGI -0.16%), 3 mg/kg (day 21, TGI 43.23%), and 8 mg/kg (day 21, TGI 106.85%) all exhibited better tumor inhibitory effects than the control molecule SGN-PDL1V (1 mg/kg, day 21, TGI -15.17%; 3 mg/kg, day 21, TGI 24.11%). MAB20-LP62 at 8 mg/kg demonstrated the strongest tumor inhibitory effect. None of the animals experienced significant body weight loss throughout the treatment period.

### Example 13. Anti-tumor effect of anti-PDL1 antibody-drug conjugate in human PDL1-positive liver cancer LD1-0011-200617 PDX xenograft NU/NU mice

A human-derived liver cancer LD1-0011-200617 model was revived in NCG mice. Once the tumor had grown to 500-800 mm³, the tumor tissue was surgically aseptically excised, and non-tumor tissues and necrotic tissues were removed. The tumor tissue was stripped and evenly cut into tissue blocks (about 30-60 mg) of about 3 mm × 3 mm × 3 mm in size, and then, which were inoculated subcutaneously on the right side of NU/NU mice. Once the average tumor volume of the mice for grouping reached 155.24 mm³, the mice were grouped randomly according to the size of the tumor volume, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume (mm3) = 1/2 × (a × b2) (wherein a represented the long diameter, and b represented the short diameter).

As shown in FIG. 11, in the PDL1-positive liver cancer PDX model LD1-0011-200617, the TGI of MAB20 at 10 mg/kg was 30.3% on day 21 post-treatment. In contrast, MAB20-LP62 (DAR8) exhibited dose-dependent tumor inhibitory effects at both doses of 3 mg/kg (day 21, TGI 75.24%) and 10 mg/kg (day 21, TGI 89.51%). The highest dose of MAB20-LP62 (DAR8) demonstrated the strongest tumor inhibitory effect. None of the animals experienced significant body weight loss throughout the treatment period.

### Example 14. Anti-tumor effect of anti-PDL1 antibody-drug conjugate in human PDL1-positive head and neck squamous carcinoma LD1-2023-411020 PDX xenograft NCG mice

A human-derived PDL1-positive head and neck squamous carcinoma LD1-2023-411020 model was revived in NCG mice. Once the tumor had grown to 500-800 mm³, the tumor tissue was surgically aseptically excised, and non-tumor tissues and necrotic tissues were removed. The tumor tissue was stripped and evenly cut into tissue blocks (about 20-30 mg) of about 3 mm × 3 mm × 3 mm in size, and then, which were inoculated subcutaneously on the right side of NCG mice. Once the average tumor volume of the mice for grouping reached 138.88 mm³, the mice were grouped randomly according to the size of the tumor volume, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume (mm3) = 1/2 × (a × b2) (wherein a represented the long diameter, and b represented the short diameter).

As shown in FIG. 12, in the anti-PD1 monoclonal antibody-resistant human PDL1-positive head and neck squamous carcinoma PDX model LD1-2023-411020, MAB20 at 10 mg/kg did not exhibit tumor inhibitory effect (day 28, TGI -34.46%) and the free loaded drug A1.3 also did not exhibit tumor inhibitory effect (day 28, TGI -8.74%); however, MAB20-LP62 (DAR8) at the same dose (day 28, TGI 102.31%) exhibited strong tumor inhibitory effect. None of the animals experienced significant body weight loss throughout the treatment period.

### Example 15. Anti-tumor effect of anti-PDL1 antibody-drug conjugate in human PDL1-positive cervical cancer LD1-0010-200614 PDX xenograft Balb/c nude mice

A human-derived PDL1-positive cervical cancer LD1-0010-200614 model was revived in Balb/c nude mice. Once the tumor had grown to 500-800 mm³, the tumor tissue was surgically aseptically excised, and non-tumor tissues and necrotic tissues were removed. The tumor tissue was stripped and evenly cut into tissue blocks (about 25-35 mg) of about 3 mm × 3 mm × 3 mm in size, and then, which were inoculated subcutaneously on the right side of Balb/c nude mice. Once the average tumor volume of the mice for grouping reached 146.74 mm3, the mice were grouped randomly according to the size of the tumor volume, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume (mm3) = 1/2 × (a × b2) (wherein a represented the long diameter, and b represented the short diameter).

As shown in FIG. 13, in the human PDL1-positive cervical cancer PDX model LD1-0010-200614, MAB20 at 10 mg/kg (day 35, TGI 67.38%) exhibited medium tumor inhibitory effect and the free loaded drug A1.3 exhibited medium tumor inhibitory effect (day 35, TGI 56.5%); however, MAB20-LP62 (DAR8) at the same dose (day 35, TGI 109.33%) exhibited strong tumor inhibitory effect. None of the animals experienced significant body weight loss throughout the treatment period.

### Example 16. Anti-tumor effect of anti-PDL1 antibody-drug conjugate in human PDL1-positive esophageal squamous carcinoma LD1-0015-362448 PDX xenograft Balb/c nude mice

A human-derived PDL1-positive esophageal squamous carcinoma LD1-0015-362448 model was revived in Balb/c nude mice. Once the tumor had grown to 500-800 mm³, the tumor tissue was surgically aseptically excised, and non-tumor tissues and necrotic tissues were removed. The tumor tissue was stripped and evenly cut into tissue blocks (about 25-35 mg) of about 3 mm × 3 mm × 3 mm in size, and then, which were inoculated subcutaneously on the right side of Balb/c nude mice. Once the average tumor volume of the mice for grouping reached 140.46 mm3, the mice were grouped randomly according to the size of the tumor volume, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume (mm3) = 1/2 × (a × b2) (wherein a represented the long diameter, and b represented the short diameter).

As shown in FIG. 14, in the human PDL1-positive esophageal squamous carcinoma PDX model LD1-0015-362448, MAB20-LP62 (DAR8) at 10 mg/kg (day 45, TGI 92.02%) exhibited strong tumor inhibitory effect. None of the animals experienced significant body weight loss throughout the treatment period.

### Example 17. Anti-tumor effect of anti-PDL1 antibody-drug conjugate in human PDL1-positive gastric cancer LD1-0017-200636 PDX xenograft NU/NU mice

A human-derived PDL1-positive gastric cancer LD1-0017-200636 model was revived in NU/NU mice. Once the tumor had grown to 500-800 mm³, the tumor tissue was surgically aseptically excised, and non-tumor tissues and necrotic tissues were removed. The tumor tissue was stripped and evenly cut into tissue blocks (about 30-60 mg) of about 3 mm × 3 mm × 3 mm in size, and then, which were inoculated subcutaneously on the right side of NU/NU mice. Once the average tumor volume of the mice for grouping reached 138.70 mm3, the mice were grouped randomly according to the size of the tumor volume, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume (mm3) = 1/2 × (a × b2) (wherein a represented the long diameter, and b represented the short diameter).

As shown in FIG. 15, in the human PDL1-positive gastric cancer PDX model LD1-0017-200636, 10 mg/kg MAB20 showed a TGI of 14.89% at day 21 and the free loaded drug A1.3 showed a TGI of 34.12%; however, 10 mg/kg MAB20-LP62 (DAR8) (day 21, TGI 108.18%) exhibited strong tumor inhibitory effect. None of the animals experienced significant body weight loss throughout the treatment period.

### Example 18. Anti-tumor effect of anti-PDL1 antibody-drug conjugate in human PDL1-positive colorectal cancer LD1-2013-362125 PDX xenograft NU/NU mice

A human-derived PDL1-positive colorectal cancer LD1-2013-362125 model was revived in NU/NU mice. Once the tumor had grown to 500-800 mm³, the tumor tissue was surgically aseptically excised, and non-tumor tissues and necrotic tissues were removed. The tumor tissue was stripped and evenly cut into tissue blocks (about 25-35 mg) of about 3 mm × 3 mm × 3 mm in size, and then, which were inoculated subcutaneously on the right side of NU/NU mice. Once the average tumor volume of the mice for grouping reached 156.99 mm3, the mice were grouped randomly according to the size of the tumor volume, ensuring similar tumor volumes between groups. The day of grouping was defined as day 0. After the start of dosing, the body weight and tumor size of the mice were measured twice a week, and the mice were dosed once a week, for three doses. Clinical symptoms observed during the experiment were recorded. The tumor volume was calculated according to the following formula: tumor volume (mm3) = 1/2 × (a × b2) (wherein a represented the long diameter, and b represented the short diameter).

As shown in FIG. 16, in the human PDL1-positive colorectal cancer PDX model LD1-2013-362125, 10 mg/kg MAB20 showed a TGI of 50.27% at day 31 and free loaded drug A1.3 showed a TGI of 37.16%; however, MAB20-LP62 (DAR8) at the same dose (TGI 101.59%) exhibited strong tumor inhibitory effect. None of the animals experienced significant body weight loss throughout the treatment period.

In addition to the depicted and claimed various embodiments, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. The above description is not intended to be exhaustive or to limit the disclosed subject matter to those embodiments disclosed.

It will be apparent to those skilled in the art that various modifications and variations can be made in the compositions and methods of the disclosed subject matter without departing from the spirit or scope of the disclosed subject matter. Thus, it is intended that the disclosed subject matter include modifications and variations that are within the scope of the appended claims and their equivalents.

Various publications, patents and patent applications are cited herein, the contents of which are hereby incorporated by reference in their entireties.

## Claims

1. An antibody-drug conjugate, a prodrug, pharmaceutically acceptable salt, or solvate thereof, or a solvate of the pharmaceutically acceptable salt, **characterized in that** the antibody-drug conjugate has a structure represented by formula (I):
TP-[L1-L2-L3-D]ₖ (I)
wherein
TP is a targeting moiety selectively binding or recognizing PDL1;
L1 is an extension unit connecting TP and L2;
L2 is an optional amino acid residue or a short peptide consisting of 2-10 amino acid residues;
L3 is a spacer element;
D is a biologically active molecule; and
k represents any numerical value between 0.1 and 10.0.

2. The antibody-drug conjugate, the prodrug, pharmaceutically acceptable salt, or solvate thereof, or the solvate of the pharmaceutically acceptable salt according to claim 1, **characterized in that** the TP is an antibody or an antigen-binding fragment thereof.

3. The antibody-drug conjugate, the prodrug, pharmaceutically acceptable salt, or solvate thereof, or the solvate of the pharmaceutically acceptable salt according to claim 2, **characterized in that** the antigen-binding fragment is selected from a full-length immunoglobulin, a single-chain Fv (scFv) fragment, an Fab fragment, an Fab' fragment, F(ab')₂, an Fv fragment, a disulfide-stabilized Fv fragment (dsFv), (dsFv)₂, an Fv-Fc fusion, an scFv-Fc fusion, an scFv-Fv fusion, a diabody, a tribody, a tetrabody, or any combination thereof.

4. The antibody-drug conjugate, the prodrug, pharmaceutically acceptable salt, or solvate thereof, or the solvate of the pharmaceutically acceptable salt according to any one of the preceding claims, **characterized in that** the TP is a humanized antibody or an antigen-binding fragment thereof.

5. The antibody-drug conjugate, the prodrug, pharmaceutically acceptable salt, or solvate thereof, or the solvate of the pharmaceutically acceptable salt according to any one of the preceding claims, **characterized in that** the TP comprises a heavy chain variable region (VH) and a light chain variable region (VL), wherein the heavy chain variable region (VH) comprises heavy chain complementarity determining region 1 (HCDR1), HCDR2, and HCDR3 contained in SEQ ID NO: 7, and the light chain variable region (VL) comprises light chain complementarity determining region 1 (LCDR1), LCDR2, and LCDR3 contained in SEQ ID NO: 8.

6. The antibody-drug conjugate, the prodrug, pharmaceutically acceptable salt, or solvate thereof, or the solvate of the pharmaceutically acceptable salt according to claim 5, **characterized in that** the amino acid sequence of HCDR1 is SEQ ID NO: 1, the amino acid sequence of HCDR2 is SEQ ID NO: 2, and the amino acid sequence of HCDR3 is SEQ ID NO: 3; and the amino acid sequence of LCDR1 is SEQ ID NO: 4, the amino acid sequence of LCDR2 is SEQ ID NO: 5, and the amino acid sequence of LCDR3 is SEQ ID NO: 6.

7. The antibody-drug conjugate, the prodrug, pharmaceutically acceptable salt, or solvate thereof, or the solvate of the pharmaceutically acceptable salt according to any one of the preceding claims, **characterized in that** the TP comprises an Fc sequence of human IgG.

8. The antibody-drug conjugate, the prodrug, pharmaceutically acceptable salt, or solvate thereof, or the solvate of the pharmaceutically acceptable salt according to any one of the preceding claims, **characterized in that** the IgG is selected from IgG1, IgG2, IgG3, and IgG4.

9. The antibody-drug conjugate, the prodrug, pharmaceutically acceptable salt, or solvate thereof, or the solvate of the pharmaceutically acceptable salt according to any one of the preceding claims, **characterized in that** the TP comprises a heavy chain variable region (VH), and the amino acid sequence of the heavy chain variable region (VH) has at least 70%, at least 80%, at least 90%, at least 95%, at least 99%, or 100% identity to SEQ ID NO: 7.

10. The antibody-drug conjugate, the prodrug, pharmaceutically acceptable salt, or solvate thereof, or the solvate of the pharmaceutically acceptable salt according to any one of the preceding claims, **characterized in that** the TP comprises a light chain variable region (VL), and the amino acid sequence of the light chain variable region (VL) has at least 70%, at least 80%, at least 90%, at least 95%, at least 99%, or 100% identity to SEQ ID NO: 8.

11. The antibody-drug conjugate, the prodrug, pharmaceutically acceptable salt, or solvate thereof, or the solvate of the pharmaceutically acceptable salt according to any one of the preceding claims, **characterized in that** the TP comprises a heavy chain (HC), and the amino acid sequence of the heavy chain (HC) has at least 70%, at least 80%, at least 90%, at least 95%, at least 99%, or 100% identity to SEQ ID NO: 9.

12. The antibody-drug conjugate, the prodrug, pharmaceutically acceptable salt, or solvate thereof, or the solvate of the pharmaceutically acceptable salt according to any one of the preceding claims, **characterized in that** the TP comprises a light chain (LC), and the amino acid sequence of the light chain (LC) has at least 70%, at least 80%, at least 90%, at least 95%, at least 99%, or 100% identity to SEQ ID NO: 10.

13. The antibody-drug conjugate, the prodrug, pharmaceutically acceptable salt, or solvate thereof, or the solvate of the pharmaceutically acceptable salt according to any one of the preceding claims, **characterized in that** the TP is a multispecific antibody.

14. The antibody-drug conjugate, the prodrug, pharmaceutically acceptable salt, or solvate thereof, or the solvate of the pharmaceutically acceptable salt according to any one of the preceding claims, **characterized in that** the TP is an afucosylated antibody.

15. The antibody-drug conjugate, the prodrug, pharmaceutically acceptable salt, or solvate thereof, or the solvate of the pharmaceutically acceptable salt according to any one of the preceding claims, **characterized in that** the Fc region comprises a C-terminal lysine.

16. The antibody-drug conjugate, the prodrug, pharmaceutically acceptable salt, or solvate thereof, or the solvate of the pharmaceutically acceptable salt according to any one of the preceding claims, **characterized in that** the Fc region comprises a deletion of the C-terminal lysine.

17. The antibody-drug conjugate, the prodrug, pharmaceutically acceptable salt, or solvate thereof, or the solvate of the pharmaceutically acceptable salt according to any one of the preceding claims, **characterized in that** the L1 is selected from 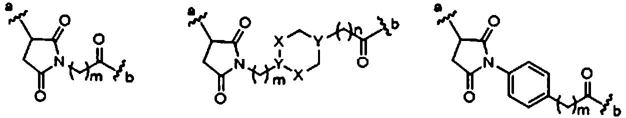 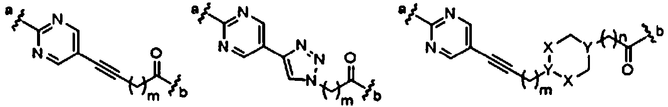 wherein L1 is connected to TP at position a via an S atom and to L2 at position b, m is any integer between 1 and 10, n is any integer between 1 and 10, and X and Y are each independently C, O, S, or N.

18. The antibody-drug conjugate, the prodrug, pharmaceutically acceptable salt, or solvate thereof, or the solvate of the pharmaceutically acceptable salt according to any one of the preceding claims, **characterized in that** the L1 is 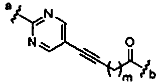
wherein m is selected from 1, 2, 3, 4, or 5,
wherein L1 is connected to TP at position a via an S atom and to L2 at position b.

19. The antibody-drug conjugate, the prodrug, pharmaceutically acceptable salt, or solvate thereof, or the solvate of the pharmaceutically acceptable salt according to any one of the preceding claims, **characterized in that** the L1 is 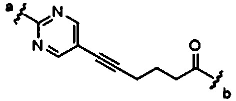 wherein L1 is connected to TP at position a via an S atom and to L2 at position b.

20. The antibody-drug conjugate, the prodrug, pharmaceutically acceptable salt, or solvate thereof, or the solvate of the pharmaceutically acceptable salt according to any one of the preceding claims, **characterized in that** the L2 is selected from 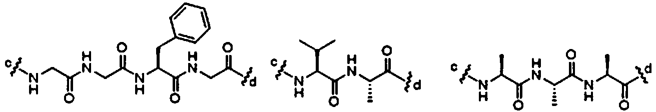 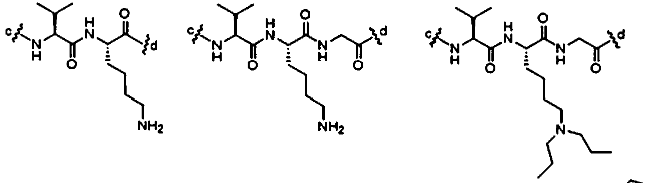 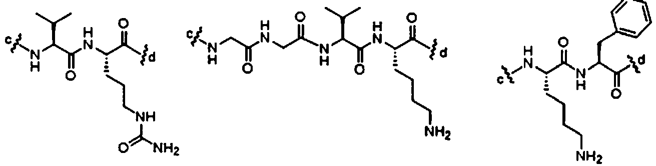 wherein L2 is connected to L1 at position c and to L3 at position d.

21. The antibody-drug conjugate, the prodrug, pharmaceutically acceptable salt, or solvate thereof, or the solvate of the pharmaceutically acceptable salt according to any one of the preceding claims, **characterized in that** the L2 is 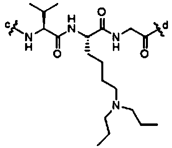 wherein L2 is connected to L1 at position c and to L3 at position d.

22. The antibody-drug conjugate, the prodrug, pharmaceutically acceptable salt, or solvate thereof, or the solvate of the pharmaceutically acceptable salt according to any one of the preceding claims, **characterized in that** the L3 is a single bond or a group selected from: 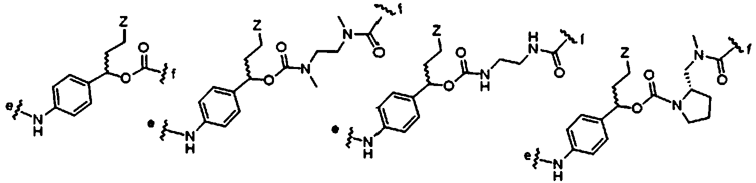
wherein L3 is connected to L2 at position e and to D at position f,
Z is selected from hydrogen, halogen, C₁-C₂₀ alkyl, C₁-C₂₀ haloalkyl, cyano, sulfo, carboxyl, C₁-C₂₀ alkoxy, C₂-C₁₀ unsaturated alkyl, a natural amino acid, an unnatural amino acid, and a short peptide consisting of the above amino acids.

23. The antibody-drug conjugate, the prodrug, pharmaceutically acceptable salt, or solvate thereof, or the solvate of the pharmaceutically acceptable salt according to any one of the preceding claims, **characterized in that** Z is selected from: 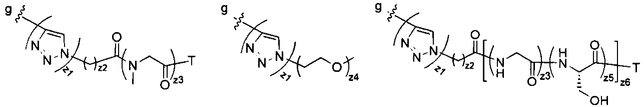 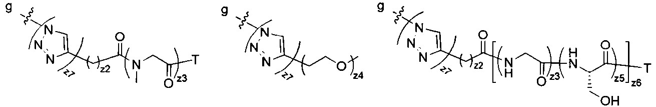 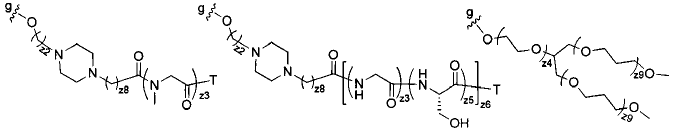 wherein Z is connected to the remainder of L3 at position g, z1-z9 are each independently an integer between 0 and 10, and T is NH₂ or OH.

24. The antibody-drug conjugate, the prodrug, pharmaceutically acceptable salt, or solvate thereof, or the solvate of the pharmaceutically acceptable salt according to any one of the preceding claims, **characterized in that** D is a cytotoxic agent.

25. The antibody-drug conjugate, the prodrug, pharmaceutically acceptable salt, or solvate thereof, or the solvate of the pharmaceutically acceptable salt according to any one of the preceding claims, **characterized in that** the D is selected from 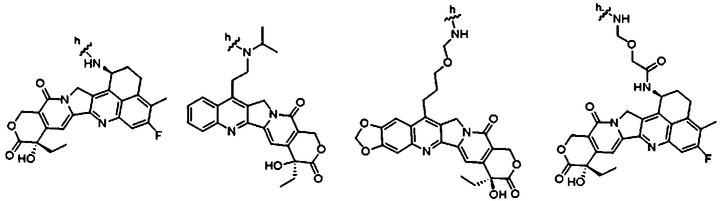 wherein D is connected to L3 at position h.

26. The antibody-drug conjugate, the prodrug, pharmaceutically acceptable salt, or solvate thereof, or the solvate of the pharmaceutically acceptable salt according to any one of the preceding claims, **characterized in that** the antibody-drug conjugate is selected from: 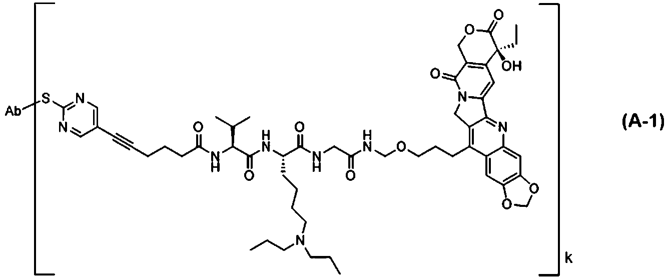 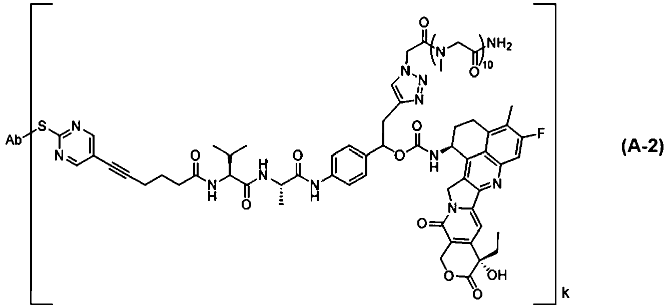 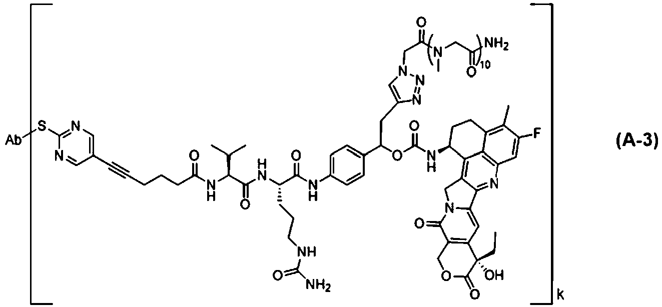 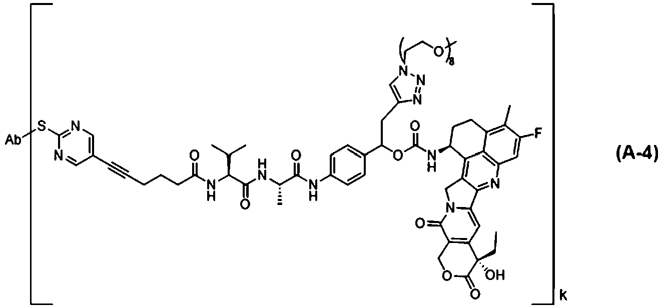 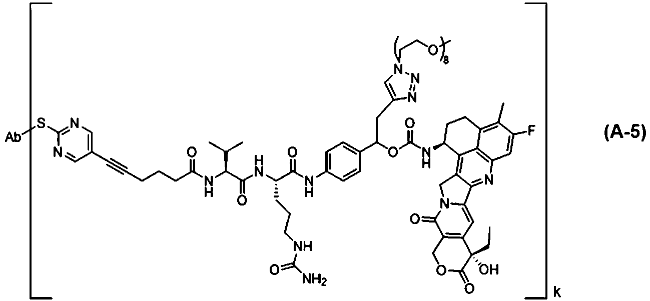 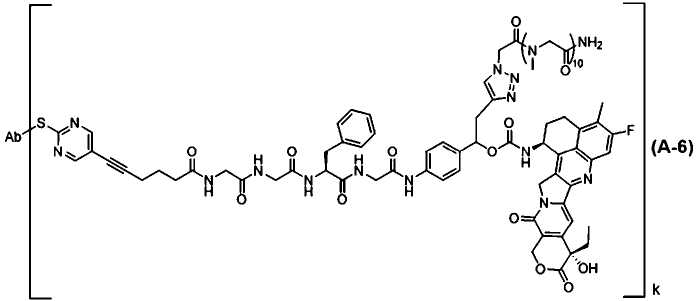 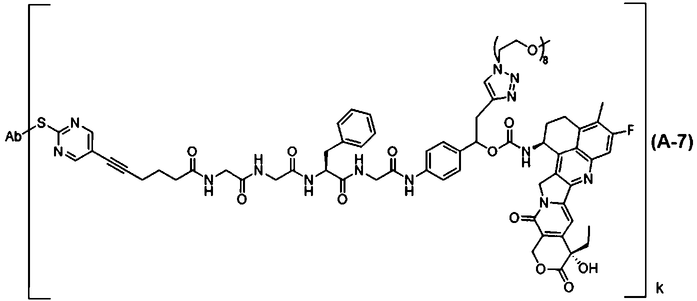 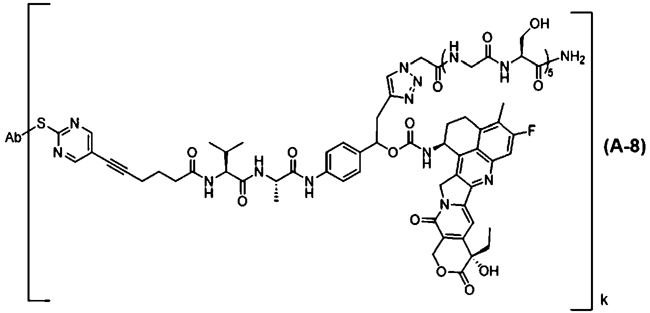 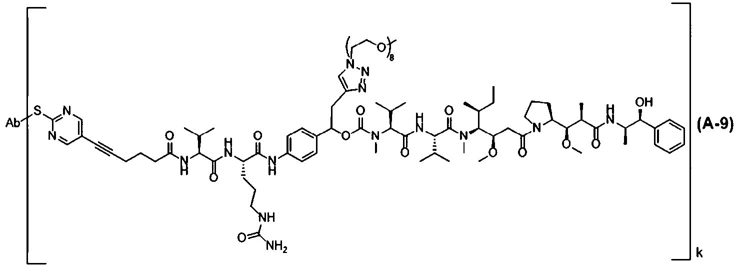 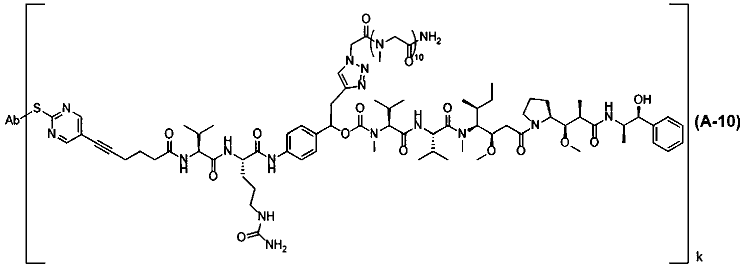 wherein Ab represents the antibody or the antigen-binding fragment thereof.

27. The antibody-drug conjugate, the prodrug, pharmaceutically acceptable salt, or solvate thereof, or the solvate of the pharmaceutically acceptable salt according to any one of the preceding claims, **characterized in that** k is about 4.0 to about 8.0, preferably k is about 4.0, about 6.0, or about 8.0.

28. A pharmaceutical composition, **characterized by** comprising the antibody-drug conjugate, the prodrug, pharmaceutically acceptable salt, or solvate thereof, or the solvate of the pharmaceutically acceptable salt according to any one of claims 1-27, and optionally, a pharmaceutically acceptable carrier, diluent, or excipient.

29. The antibody-drug conjugate, the prodrug, pharmaceutically acceptable salt, or solvate thereof, or the solvate of the pharmaceutically acceptable salt according to any one of claims 1-27 or the pharmaceutical composition according to claim 28, for use as a drug.

30. The antibody-drug conjugate, the prodrug, pharmaceutically acceptable salt, or solvate thereof, or the solvate of the pharmaceutically acceptable salt according to any one of claims 1-27 or the pharmaceutical composition according to claim 28, for use in the treatment or prevention of a tumor or cancer.

31. The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 30, **characterized in that** the tumor or cancer is a tumor or cancer related to PDL1 expression.

32. The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 30, **characterized in that** the tumor or cancer is selected from melanoma, lung cancer, head and neck cancer, breast cancer, ovarian cancer, urothelial cancer, hepatocellular carcinoma (HCC), gastric cancer, cervical cancer, colon cancer, rectal cancer, colorectal cancer, bladder cancer, esophageal cancer, kidney cancer, prostate cancer, pancreatic cancer, and glioma.

33. The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 32, **characterized in that** the lung cancer is non-small cell lung cancer (NSCLC) or small cell lung cancer (SCLC).

34. The antibody-drug conjugate, the prodrug, pharmaceutically acceptable salt, or solvate thereof, or the solvate of the pharmaceutically acceptable salt, or the pharmaceutical composition for use in the treatment or prevention of a tumor or cancer according to claim 32, **characterized in that** the breast cancer is triple negative breast cancer (TNBC).

35. An article of manufacture or kit, **characterized by** comprising the antibody-drug conjugate, the prodrug, pharmaceutically acceptable salt, or solvate thereof, or the solvate of the pharmaceutically acceptable salt according to any one of claims 1-27 or the pharmaceutical composition according to claim 28, and instructions for use.

36. A method for preventing or treating a tumor or cancer, **characterized by** comprising administering to a subject in need thereof a prophylactically or therapeutically effective amount of the antibody-drug conjugate, the prodrug, pharmaceutically acceptable salt, or solvate thereof, or the solvate of the pharmaceutically acceptable salt according to any one of claims 1-27 or the pharmaceutical composition according to claim 28.

37. The method according to claim 36, **characterized in that** the tumor or cancer is a tumor or cancer related to PDL1 expression.

38. The method according to claim 36, **characterized in that** the tumor or cancer related to PDL1 expression is selected from melanoma, lung cancer, head and neck cancer, breast cancer, ovarian cancer, urothelial cancer, hepatocellular carcinoma (HCC), gastric cancer, cervical cancer, colon cancer, rectal cancer, colorectal cancer, bladder cancer, esophageal cancer, kidney cancer, prostate cancer, pancreatic cancer, and glioma.

39. The method according to claim 38, **characterized in that** the lung cancer is non-small cell lung cancer (NSCLC) or small cell lung cancer (SCLC).

40. The method according to claim 38, **characterized in that** the breast cancer is triple negative breast cancer (TNBC).

41. The method according to any one of claims 36-40, **characterized in that** the method comprises administering to the subject an additional therapeutic agent selected from an anti-tumor agent, a chemotherapeutic agent, a growth inhibitor, and a cytotoxic agent.

42. The method according to any one of claims 36-41, **characterized in that** the method comprises further administering to the subject a radiotherapy.

43. Use of the antibody-drug conjugate, the prodrug, pharmaceutically acceptable salt, or solvate thereof, or the solvate of the pharmaceutically acceptable salt according to any one of claims 1-27 or the pharmaceutical composition according to claim 28 in the preparation of a medicament for use in the prevention or treatment of a tumor or cancer.

44. Use of the antibody-drug conjugate, the prodrug, pharmaceutically acceptable salt, or solvate thereof, or the solvate of the pharmaceutically acceptable salt according to any one of claims 1-27 or the pharmaceutical composition according to claim 28 in combination with an additional therapeutic agent in the preparation of a medicament for use in the prevention or treatment of a tumor or cancer.

45. The use according to claim 43 or 44, **characterized in that** the tumor or cancer is a tumor or cancer related to PDL1 expression.

46. The use according to claim 45, **characterized in that** the tumor or cancer related to PDL1 expression is selected from melanoma, lung cancer, head and neck cancer, breast cancer, ovarian cancer, urothelial cancer, hepatocellular carcinoma (HCC), gastric cancer, cervical cancer, colon cancer, rectal cancer, colorectal cancer, bladder cancer, esophageal cancer, kidney cancer, prostate cancer, pancreatic cancer, and glioma.

47. The use according to claim 46, **characterized in that** the lung cancer is non-small cell lung cancer (NSCLC) or small cell lung cancer (SCLC).

48. The use according to claim 46, **characterized in that** the breast cancer is triple negative breast cancer (TNBC).

49. The use according to any one of claims 44-48, **characterized in that** the additional therapeutic agent is selected from an anti-tumor agent, a chemotherapeutic agent, a growth inhibitor, and a cytotoxic agent.
